# EUROPEAN PATENT SPECIFICATION

(11) **EP 2 770 898 B1**
(45) Date of publication and mention of the grant of the patent: **26.07.2017**
(21) Application number: 12784824.0
(22) Date of filing: 24.10.2012
(51) Int. Cl.: A61B 1/12, A61B 17/34

(54) **JAWED TROCAR TIP ASSEMBLY**
TROKARSPITZENANORDNUNG MIT BACKEN
ENSEMBLE POINTE DE TROCART À MÂCHOIRES

(30) Priority: 24.10.2011 US 201113280233; 08.12.2011 US 201161568623 P; 02.02.2012 US 201261593957 P; 16.04.2012 US 201261624963 P
(43) Date of publication of application: 03.09.2014
(73) Proprietor: Facseal LLC, Houston, TX 77002 (US)
(72) Inventor: FEUER, Gerald, Atlanta, GA 30327 (US); FOSTER, Clark, B., Mission Viejo, CA 92692 (US); SANDERS, Gerald, Jay, Palo Alto, CA 94301 (US)
(74) Representative: Gill Jennings & Every LLP
(86) International application number: PCT/US2012/061741
(87) International publication number: WO 2013/063153

(56) References cited:
- EP-A2- 1 210 904
- EP-A2- 1 854 421
- EP-A2- 2 111 782
- WO-A1-92/12680
- WO-A1-96/23536
- US-A1- 2006 079 925
- US-A1- 2010 022 958

## Description

### FIELD OF TECHNOLOGY

The present disclosure relates generally to trocar devices, and more specifically, to jawed trocar assemblies which can be utilized in surgical procedures. A direct vision dissecting port for providing safe entry into a body cavity is disclosed in US A 2006/0079925.

### BRIEF DESCRIPTION OF THE DRAWINGS

Implementations of the present application will now be described, by way of example only, with reference to the attached Figures, wherein:
Figure 1 is a perspective view of an endoscopic tool assembly in accordance with an exemplary embodiment of the present disclosure;
Figure 2 is a perspective view of a locking member for a jawed trocar assembly in accordance with an exemplary embodiment of the present disclosure;
Figure 3 is a partial side view of a jawed trocar assembly illustrating an insertion of an implement into the jawed trocars in accordance with an exemplary embodiment of the present disclosure having an implement inserted therethrough;
Figure 4 is a side view of a second end of a jawed trocar assembly in a first position (for example, a rest position) in accordance with an exemplary embodiment of the present disclosure;
Figure 5 is a side view of a second end of a jawed trocar assembly in a second position (for example, an expanded position) in accordance with an exemplary embodiment of the present disclosure;
Figure 6 is a side view of another exemplary embodiment of a jawed trocar assembly in a first position (such as a rest position) and illustrating an implement (such as an endoscope) to be inserted therein in accordance with the present disclosure;
Figure 7 is a perspective view of a side of the jawed trocar assembly illustrated in Figure 6 except in a second position (such as an expanded position);
Figure 8 is a perspective view of a locking member configured for engagement with the jawed trocar assembly to maintain the jawed trocar assembly in the rest position in accordance with the present disclosure;
Figures 9-13 are cross-sectional views of an exemplary embodiment of a jawed trocar assembly, in accordance with the present disclosure, having a scope cleaner mechanism coupled thereto, illustrating several positions of the scope cleaner mechanism against an endoscope inserted into the jawed trocar assembly to clean debris from the surface of the endoscope;
Figures 14 and 15 are perspective views of another exemplary embodiment of a jawed trocar assembly, in accordance with the present disclosure, having a scope cleaner mechanism which is rotatable;
Figures 16-18 are perspective views of another embodiment of a scope cleaner mechanism which can be inserted into a jawed trocar assembly in accordance with the present disclosure;
Figure 19 is a perspective view of an exemplary embodiment of a jawed trocar assembly in accordance with the present disclosure having a biasing mechanism configured to bias the jawed trocar assembly in the second position, for example the expanded position, wherein the illustrated jawed trocar assembly is in the first position, for example, the rest position;
Figure 20 is a perspective view of the jawed trocar assembly illustrated in Figure 19, wherein the biasing mechanism has been actuated to bias the jawed trocar assembly in the expanded position;
Figure 21 is a cross-sectional view of the jawed trocar assembly illustrated in Figure 19 and 20 illustrating an actuator of the biasing mechanism;
Figure 22 is a perspective view of a reflecting member;
Figure 23 is a cross-sectional view of the reflecting member assembled with a jawed trocar assembly in accordance with an exemplary embodiment;
Figure 24 is a perspective view of a removal trocar assembly in accordance with an exemplary embodiment;
Figure 25 is an exploded view of a trocar assembly having a trocar and a tip assembly for the trocar in accordance with an exemplary embodiment;
Figure 26 is a perspective view of a portion of a trocar assembly assembled with a tip assembly in accordance with an exemplary embodiment showing the interconnections of components of the tip assembly and the trocar assembly in phantom;
Figures 27-29 are perspective views of a trocar assembly illustrating the removal of a locking member in accordance with an exemplary embodiment;
Figures 30-31 are cross-sectional views of a tip assembly illustrating the coupling of a jaw assembly component and adapter sleeve of the tip assembly in accordance with an exemplary embodiment;
Figures 32-35 are cross-sectional views of a tip assembly coupled with a trocar assembly illustrating the removal of a locking member of the tip assembly;
Figure 36 is an exploded view of a tip assembly for a trocar assembly in accordance with an exemplary embodiment;
Figures 37-38 are cross-sectional views of the tip assembly illustrated in Figure 36 illustrating the coupling of the jaw assembly component and the adapter sleeve of the tip assembly illustrated in Figure 36;
Figures 39-42 are cross-sectional views of a tip assembly illustrated in Figures 78-38 coupled with a trocar assembly and illustrating the removal of a locking member of the tip assembly;
Figure 43 is a side elevation view of an exemplary tip assembly, in a closed configuration, having an exemplarily jaw retention device in the form of a band;
Figure 44 is a side elevation view of the exemplary tip assembly of Figure 43, in an open configuration, wherein the band has been fractured;
Figure 45 is a side elevation view of an exemplary tip assembly, in a closed configuration, having an exemplarily jaw retention device in the form of at least one tab;
Figure 46 is a side elevation view of the exemplary tip assembly of Figure 45, in an open configuration, wherein the at least one tab has been fractured;
Figure 47A is a top view of an exemplarily scope cleaner having a groove formed therein, according to an exemplarily embodiment;
Figure 47B is a cross-sectional view of the scope cleaner of Figure 47A taken along line B-B;
Figure 48A is a top view of another exemplarily scope cleaner having a groove formed therein, according to an exemplarily embodiment;
Figure 48B is a cross-sectional view of the scope cleaner of Figure 48A taken along line B-B;
Figure 49A is a top view of yet another exemplarily scope cleaner having a groove formed therein, according to an exemplarily embodiment; and
Figure 49B is a cross-sectional view of the scope cleaner of Figure 49A taken along line B-B.
Figure 50 is a perspective view of yet another exemplarily tip assembly in an open configuration, according to an exemplarily embodiment;
Figure 51 is a cross-sectional view of the tip assembly of Figure 50;
Figure 52 is a a perspective view of still another exemplarily tip assembly in an open configuration, according to an exemplarily embodiment;
Figure 53 is a cross-sectional view of the tip assembly of Figure 52.

### DETAILED DESCRIPTION

The invention is defined in claim 1. It will be appreciated that for simplicity and clarity of illustration, where appropriate, reference numerals have been repeated among the different figures to indicate corresponding or analogous elements. In addition, numerous specific details are set forth in order to provide a thorough understanding of the implementations described herein. However, it will be understood by those of ordinary skill in the art that the implementations described herein can be practiced without these specific details. In other instances, methods, procedures and components have not been described in detail so as not to obscure the related relevant feature being described. Also, the description is not to be considered as limiting the scope of the implementations described herein.

Several definitions that apply throughout this document will now be presented. The phrase "coupled" is defined as connected, whether directly or indirectly through intervening components and is not necessarily limited to physical connections.

Medical procedures performed within the body cavity of a patient are typically achieved through narrow tubes or cannulas inserted through a small entrance incision in the skin of the patient. Typically, the cannula is extended into the patient's body through the entranceincision to provide an access port. The access port allows the surgeon to insert a number of different medical implements therethrough. For example, the medical implements can be inserted through the cannula or a trocar to access portions of the body cavity that are far removed from the incision. Medical procedures which utilize cannulas and/or trocars can include endoscopic procedures in which an endoscope is inserted into the cavity to provide the surgeon with a view of the interior cavity of the patient, laparoscopic procedures, colonoscopic procedures, and other minimally invasive procedures which are performed via small incisions in the skin of a patient. Many of these procedures are often performed remotely from the incision. Consequently, application of the medical implements can be complicated by a reduced field of view and/or a reduced tactile feedback from the surgeon at the proximal end of the medical implement.

A trocar assembly in accordance with the present disclosure can include a hollow elongate member and a jaw. The hollow elongate member can have a first end a second end. The first end can be open and configured to receive an implement insertable therethrough. The jaw can be hingedly coupled to the second end of the hollow elongate member. The jaw can be adapted to penetrate at least one layer of a body tissue. For example, the jaw can be adapted to penetrate at least one layer of a body tissue. The trocar assembly can have a first position and a second position. The first position can be a rest position wherein the jaw is substantially parallel to a longitudinal axis of the hollow elongate member. The second position can be an expanded position wherein the jaw is rotated such that an end of the jaw is positioned radially away from the longitudinal axis. In the event the implement is inserted through the hollow elongate member, when the trocar assembly is placed in the second position, the implement can be protrudable therefrom. For example, in the event the implement is inserted and received within the hollow elongate member, the trocar assembly can be transitionable into the second position. For example, when the implement is longitudinally advanced through the hollow elongate member to protrude out from the second end of the hollow elongate member, the jaw can be rotated such that the end of the jaw is positioned radially away from the longitudinal axis of the hollow elongate member, thereby placing the trocar assembly in the second position. In this second position, as the implement is protrudable from the second end of the hollow elongate member and from the end of the jaw, the implement can engage portions of a body cavity of a patient to perform medical procedures within the body cavity.

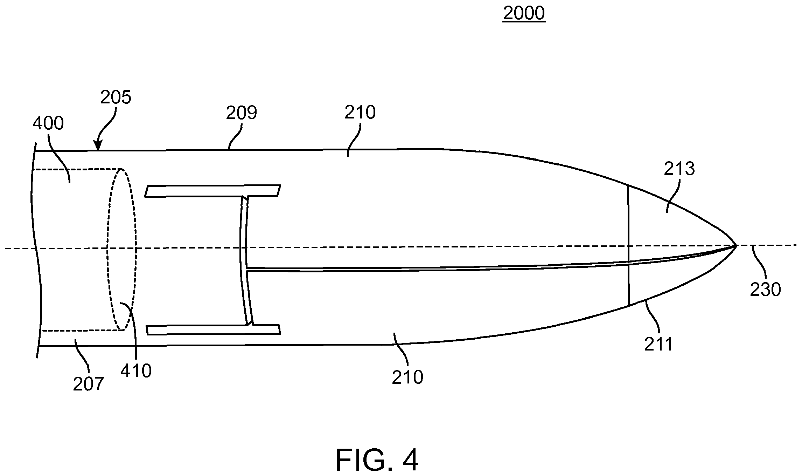

Other configurations and arrangements will be described below in relation to illustrated implementations. One of ordinary skill would appreciate that the elements from the illustrated implementations can be optionally included, combined, omitted, and arranged in various combinations to achieve the described benefits of the presently disclosed notification device. It will also be appreciated that while Figures 4-23 illustrate distal ends of the jawed trocar assembly, these views are merely for illustration, and those of ordinary skill in the art will appreciate that the length of the jawed trocar assembly can vary from views illustrated therein.

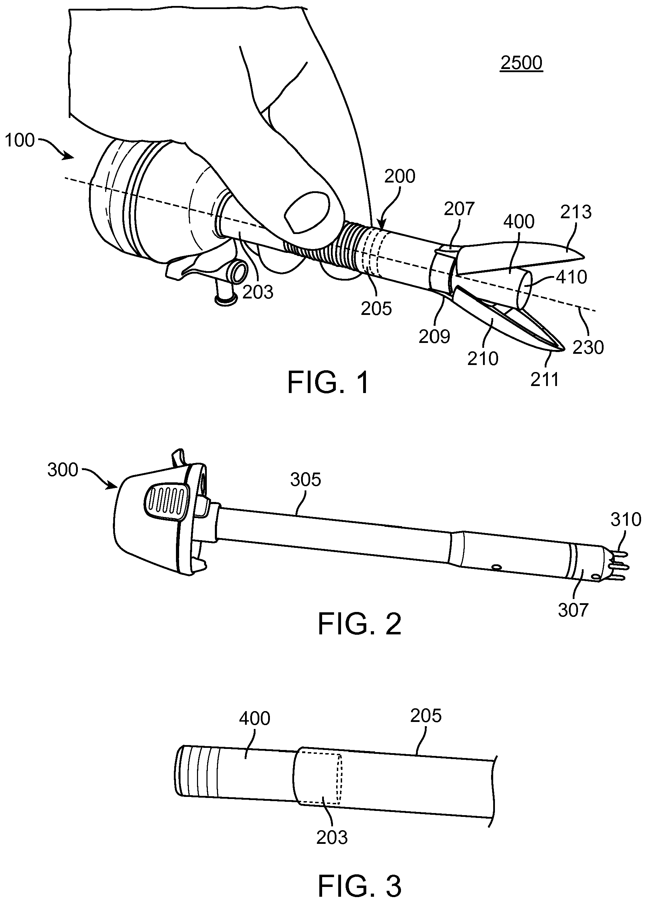

Figure 1 is perspective view of an exemplary embodiment of a medical implement assembly having a trocar assembly in accordance with the present disclosure. The medical implement assembly 100 in Figure 1 can include a trocar assembly 200 and an implement 400. For example, in Figure 1, the implement 400 is an endoscope. However, the implement 400 can also be a laparoscope, an endoscopic stapler, a suctioning device, a fluid line, or other implement. The trocar assembly 200 can include a hollow elongate member 205 having a first end 203 and a second end 209. The first end 203 can be the proximal end which is closest to the surgeon during medical procedures. The implement 400 can be insertable through the first end 203 of the hollow elongate member 205, as shown in Figure 3. The second end 207 can be the distal end which is inserted into the body cavity of a patient for medical procedures. The second end 207 can also have a circumference 209. As illustrated in Figure 1, the trocar assembly 200 can include at least one jaw 210 coupled to the second end 209 of the elongate member 205. For example, in Figure 1, the at least one jaw 210 is hingedly coupled to a circumference 209 of the second end 207 of the hollow elongate member 205. The trocar assembly 200 can have a first position 2000 (shown in Figure 4) to a second position 2500 (shown in Figure 1). In the second position 2500, shown as an expanded position in Figure 1, the at least one jaw 210 can be rotated such than an end 213 of the at least one jaw 210 is positioned radially away from the longitudinal axis 230 of the hollow elongate member 205. Those of ordinary skill in the art will appreciate that while Figure 1 illustrates the at least one jaw 210 rotates radially away from the longitudinal axis, the at least one jaw 210 can rotate along an axis of rotation offset from the longitudinal axis of the hollow elongate member 205. For example, the at least one jaw 210 can be hinged to the circumference of the hollow elongate member 205 such that the at least one jaw 210 can swing away from the longitudinal axis 230 of the hollow elongate member 205, similar to the way a door moves on a door hinge.

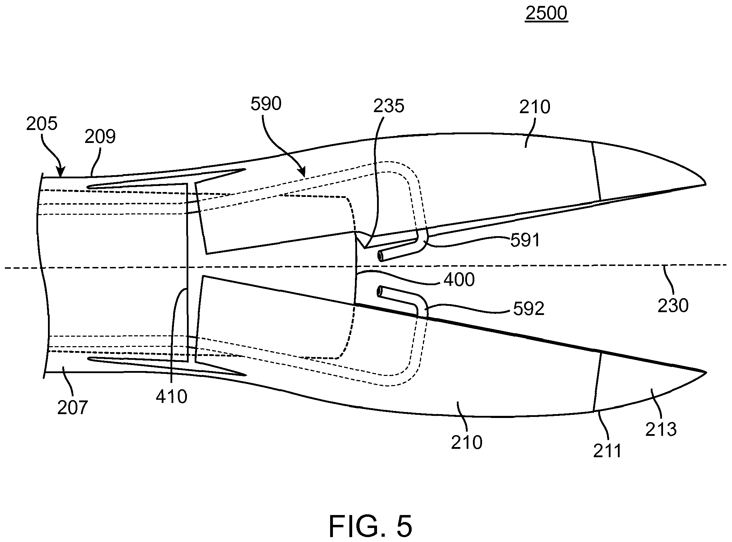

In Figure 1, the at least one jaw 210 includes a pair of jaws. Each of the pair of jaws 210 can be hingedly coupled to the second end 207 of the hollow elongate member 205. For example, in Figure 1, each jaw is hingedly coupled to the circumference 209 of the second end 207 of the hollow elongate member 205. Also illustrated in Figure 1, the pair of jaws 210 can be coupled to the hollow elongate member 205 such that each jaw 210 is opposite to the other. In at least one embodiment, at least one of the pair of jaws 210 can have a penetrating surface 211 adapted to penetrate through at least one layer of the body tissue. In another embodiment, both of the jaws 210 can have a penetrating surface 211. The penetrating surface 211 can be an abrasive surface, a smooth surface, a blade, a razor, a sharp edge, or other surface which allows the at least one jaw 210 to penetrate through at least one layer of body tissue. Further details as to the first position 2000 and second position 2500 of a trocar assembly 200 having a pair of jaws 210 will be discussed below with respect to Figures 4 and 5. In this second position 2500, the implement 400 can protrude from the second end 207 of the hollow elongate member, through the jaws 210, and out from the jaws 210. For example, in the second position 2500, the distal end 410 of the implement 300 can protrude form the end 213 of the at least one jaw 210.

In Figure 1, the at least one jaw 210 can be configured to penetrate (for example, make an incision) through at least one layer of a body tissue. Those of skill will appreciate that in order to penetrate at least one layer of body tissue, the at least one jaw 210 of the trocar assembly 200 can be substantially rigid to allow pressure to be placed on the trocar assembly 200 to penetrate the at least one layer of body tissue. For example, the at least one jaw 210 can be made of a rigid material. For example, the jaw can be made of a hard plastic, metal, composite material, or other material that does not substantially deform when pressure is applied to the at least one jaw 210 to penetrate through at least one layer of body tissue. In another embodiment, the at least one jaw 210 can be made of a semi-deformable material, such as a pliable plastic or other semi-deformable material that does not substantially deform when pressure is applied to the at least one jaw 210 to penetrate through the at least one layer of body tissue. Those of ordinary skill in the art will appreciate that the at least one jaw 210 can be made of a biologically-safe material, as the at least one jaw 210 is insertable into a body cavity of patient. Some examples of biologically-safe material include but are not limited to polycarbonate and polysuphone.

In another embodiment, the at least one jaw 210 can be made of a deformable plastic, but the at least one jaw 210 can be couplable to a locking member 300 (shown in Figure 2) which when coupled to the at least one jaw 210, provides the at least one jaw 210 with a rigidity that reduces the deformation of the at least one jaw 210 when pressure is applied thereto to penetrate through the at least one layer of body tissue. For example, in an embodiment where the at least one jaw 210 includes a pair of jaws, a locking member 300 (shown in Figure 2) can be coupled to the pair of jaws to maintain the pair of jaws in the rest position 2000 (shown in Figure 4) and to provide the pair of jaws with sufficient rigidity such that when pressure is placed on the jaws 210 to penetrate through a layer of body tissue, the pair of jaws 210 will not deform. Figure 2 illustrates an exemplary locking member 300 which can be couplable to a pair of jaws. For example, in Figure 2, the locking member 300 can include a rod 305. The rod 305 can be a tube, a hollow tube, a cylindrical member, a hollow cylindrical member, a wire, or any other structure or member which can be coupled to the hollow elongate member 205 and can maintain the jaw in the rest position. In Figure 2, the rod 305 can be insertable through the hollow elongate member 205. At least one protrusion 310 can coupled to an end 307 of the rod 305. For example, the at least one protrusion 310 can be welded, screwed, glued, or otherwise attached to the rod 305. In other embodiments, the at least on protrusion 310 can be formed at the end of the rod 305.

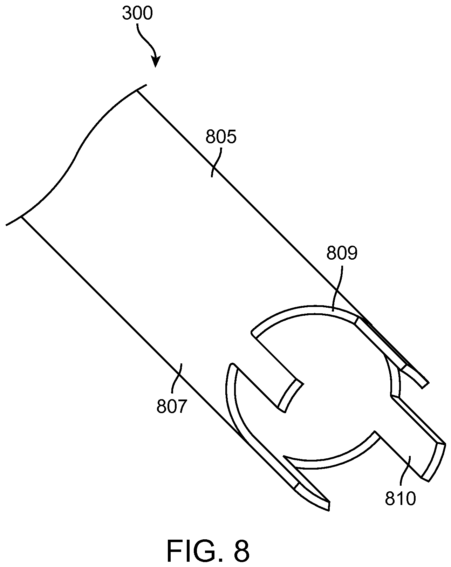

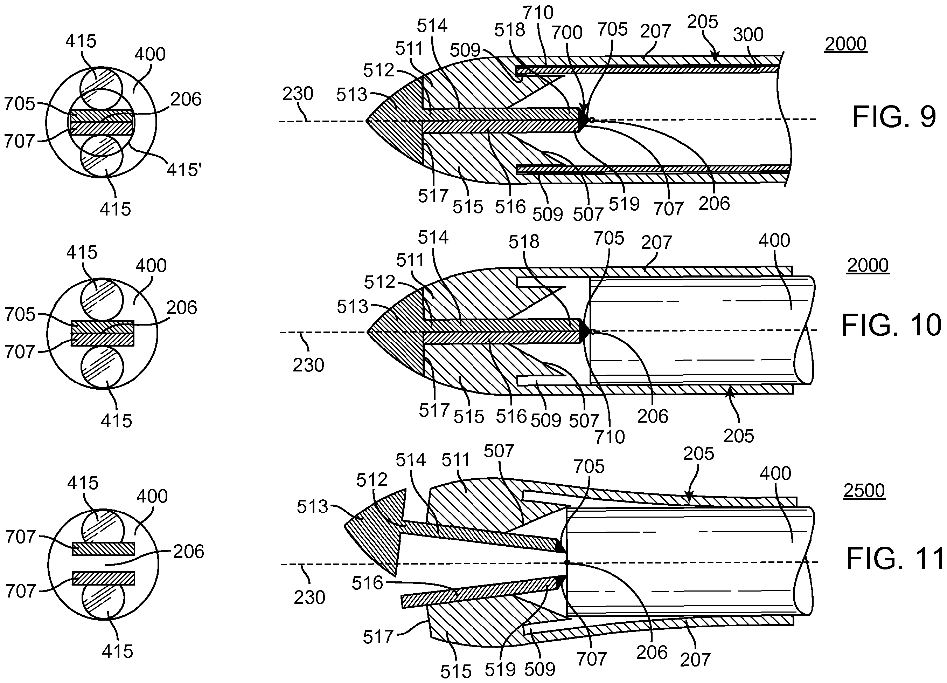

In Figure 2, the at least one protrusion 310 can be coupled to the end 307 of the rod 305 that is adjacent the at least one jaw 210 coupled to the hollow elongate member 205, in the event the locking member 300 is inserted into the hollow elongate member. In Figure 2, the locking member 300 includes four protrusions 310. However, those of ordinary skill in the art will appreciate that any number of protrusions 310 can be implemented. Each of the protrusions 310 can matingly engage with a corresponding recess (not shown) formed in at least one of the jaws 210 illustrated in Figure 1. In the event the locking member 100 is inserted into the hollow elongate member 205 and at least one of the protrusions 310 matingly engages the corresponding recess, the jaws 210 can be locked or maintained in the rest position 2500, thereby allowing pressure to be placed on the jaws 210 to penetrate through at least one layer of body tissue, without substantially deforming the jaws 210. The locking member 300 can be removable from the hollow elongate member 205, thereby disengaging the at least one protrusion 310 from the jaws 210. In the event the locking member 300 is removed from the hollow elongate member 205, the trocar assembly 100 can be transitionable between a first position (for example, rest position) and a second position (for example, and expanded position). Figure 8 illustrates another embodiment of a locking member 300. In Figure 8, the locking member 300 includes a hollow cylindrical member 805 having an end 807. At least one tab 810 can be coupled to the end 807 of the hollow cylindrical member 805. For example, as illustrated in Figure 8, a plurality of tabs 810 are formed along the circumference 809 of the end 807 of the hollow cylindrical member 805. Similar to the protrusions of the locking member 300 illustrated in Figure 2, when the hollow cylindrical member 805 is inserted in the hollow elongate member 205 of the trocar assembly 200, the plurality of tabs 805 can engage recesses 509 (shown in Figures 9-13) formed in the at least one jaw 210 to maintain the trocar assembly 200 in a rest position 2000. For example, by maintaining the jaws 210 in the rest position 2000.

Figures 4 and 5 illustrate a partial view of the at least one jaw 210 illustrated in the trocar assembly 100 of Figure 1. Figure 4 illustrates the at least one jaw 210 the at least one jaw 210 in a first position 2000 that is a rest position. For example, in Figure 4, the at least one jaw 210 the at least one jaw 210 is a pair of jaw 210. In the first position 2000, the pair of jaws 210 are each substantially parallel to the longitudinal axis of the hollow elongate member 205. Also illustrated in Figure 4, in the first position 2000, the pair of jaws 210 are positioned with respect to one another such that the ends 213 of each of the jaws 210 form a substantially conical contour adapted to penetrate the layer of the body tissue. The first position 2000 can be a position in which an implement 400 has been inserted through the cavity of the hollow elongate member 205 but has not been advanced therethrough to protrude from the second end 207 of the hollow elongate member 205. When the implement 400 has been inserted through the cavity of the hollow elongate member 205 and is advanced therethrough such that the implement 400 begins to protrude out from the second end 207 of the hollow elongate member 205 and begins to engage an inner surface 235 (shown in Figure 5) of at least one of the jaws 210, the trocar assembly 100 can be transitioned into the second position 2500.

Figure 5 illustrates a partial view of the trocar assembly 100 as the trocar assembly is transitioning into the second position 2500. In the second position 2500, each of the jaws of the pair of jaws 210 can be rotated such that the ends 213 of each jaw 210 are positioned radially away from the longitudinal axis 230. In this second position 2500, the implement 400 can be protrudable therefrom to perform medical procedures within the body cavity. As illustrated in Figure 5, the end 410 of the implement 400 can engage an interior surface 235 of at least one of the jaws 210 as the implement is advanced through the hollow elongate member 205. As the implement 400 is further advanced through the hollow elongate member 205 to protrude out from the second end 207, the end 410 of the implement 400 can apply a force against the interior surface 235 of at least one of the jaws 210 to permit the rotation of at least one of the jaws 210 about the second end 207 of the hollow elongate member 205 to displace or position the ends 213 of the jaws 210 radially away from the longitudinal axis 230 of the hollow elongate member 205. The further the implement 400 is advanced through hollow elongate member 205, the jaws 210 are further rotated, and the radial distance between the ends 213 of the jaws 210 from the longitudinal axis 230 of the hollow elongate member 205 is increased. The implement 400 can be advanced through the hollow elongate member 205 and through the jaws 210 such that the end 410 of the implement 400 can protrude a distance away from the ends 213 of the jaws 210. As the implement 400 can protrude a distance away from the ends 213 of the jaws 210, the implement 400 can be manipulated for medical procedures within the patient's body cavity without substantial interference from the jaws 210.

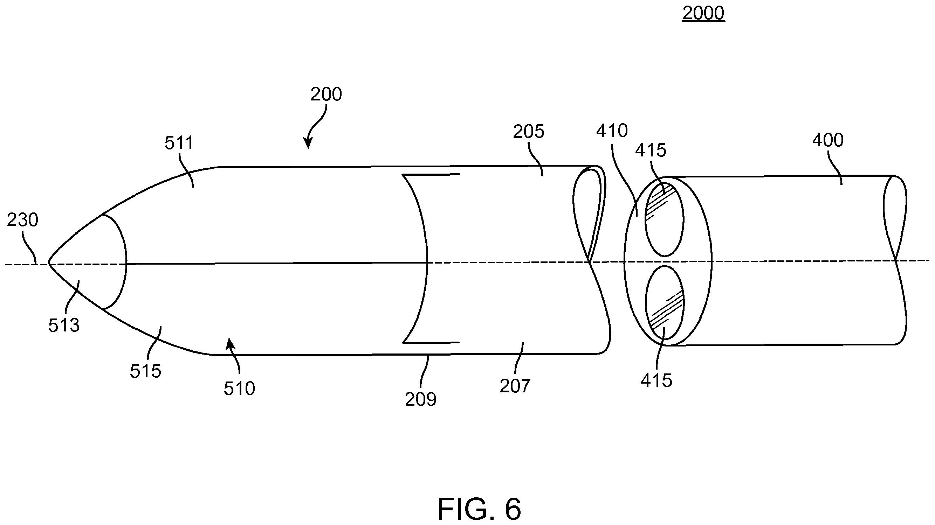

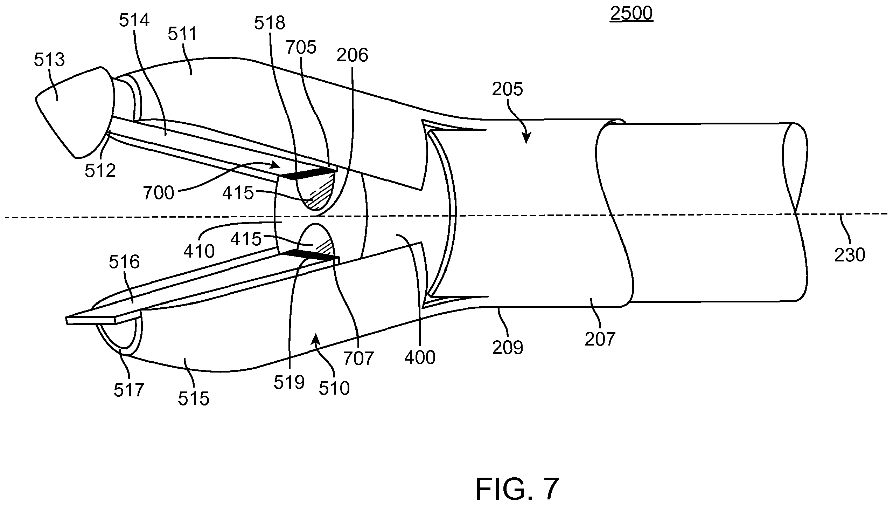

While Figures 4 and 5 illustrate a trocar assembly 200 having a pair of jaws 210 which are substantially identical, in another embodiment, the pair of jaws 210 need not be identical. For example, Figure 6 illustrates another embodiment of a trocar assembly 200 having a pair of jaws 510 has a first jaw 511 and a second jaw 515, the second jaw 510 being different from the first jaw 511. In Figure 6, the trocar assembly 200 is in the first position 2000, wherein the first jaw 511 and second jaw 515 are each substantially parallel to the longitudinal axis 230 of the hollow elongate member 250. In Figure 6, the first jaw 511 includes a penetrating member 513 at an end thereof. In Figure 6, the penetrating member 513 is configured to penetrate at least one layer of body tissue. For example, the penetrating member 513 can be a substantially conical tip, as illustrated in Figure 6, a blade, a tip having a cutting surface thereon, or any other member which can penetrate at least one layer of body tissue. In Figure 6, the second jaw 515 does not include a penetrating member. Instead the second jaw 215 is configured to be positioned adjacent the first jaw 511 in the first position 2000 such that the penetrating member 513 extends longitudinally further than an end 517 (shown in Figure 7) of the second jaw 515. For example, in Figure 6, the penetrating member 513 extends longitudinally further than the end 517 of the second jaw 515 in the first position 2000 (for example the rest position) such that the exterior surfaces of the penetrating member 513 and the second end 517 form a substantially conical contour. That is, in at least one embodiment where the first jaw 511 includes a penetrating member 513, the first jaw 511 and the second jaw 515 can be configured such that in the first position 2000 (for example, the rest position), the first jaw 511 and the second jaw 515 can form a substantially contiguous contour. With the substantially contiguous contour, the trocar assembly 205 can have a substantially uniform shape that permits a clean penetration through at least one layer of body tissue. Also illustrated in Figure 6 is an implement 400 that is an endoscope which is insertable through the hollow elongate member 205. The endoscope 400 in Figure 6 has two lenses 415. However, in other embodiments, the endoscope 400 can have one lens 415' (shown in Figure 9) or more than one lens 415. For example, in Figure 9, a lens 415' of an endoscope 400 having a single lens 415' is shown overlaid on the two lenses 415.

Figure 7 and 9-13 illustrate another embodiment of a trocar assembly 200 having a pair of jaws 511, 515. In Figure 7, the trocar assembly 200 can have a first jaw 511 having a first slider 514 axially translatably coupled thereto. For example, the first slider 514 can be configured to translate along the first jaw 511 parallel to the longitudinal axis of the first jaw 511. That is, the first slide 514 can be axially translatable with respect to the first jaw 511. The longitudinal axis of the first jaw 511 can be parallel to the longitudinal axis 230 of the hollow elongate member 205. Similarly, the second jaw 515 can have a second slider 516 axially translatably coupled thereto. The second slider 516 can be configured to translate along the longitudinal axis of the second jaw 516. That is, the second slider 516 can be axially translatable with respect to the second jaw 516. The longitudinal axis of the second jaw 516 can be parallel to the longitudinal axis 230 of the hollow elongate member 205 in the first position 2000. In Figure 7, though not illustrated, when the trocar assembly 205 is in the first position 2000 (for example, the rest position), the first slider 514 and the second slider 516 can be positioned adjacent one another parallel to the longitudinal axis 230 of the hollow elongate member 205. In at least one embodiment, the first slider 514 and the second slider 516 can be translatably coupled to their respective jaws 511, 515 via a pin and slot coupling (not shown). For example, the first slider 514 and second slider 516 can have a slot (not shown) formed therein. The slot of the first slider 514 can be configured to engage a pin (not shown) coupled to the first jaw 511. The slot of the second slider 516 can be configured to engage a pin (not shown) coupled to the second jaw 515. Thus, the pins of the first jaw 511 and second jaw 515, slide within the slots of the respective first slider 514 and second slider 516, thereby providing for the axially translatable cooperation between the jaws 511, 515, and the sliders 514, 516. Those of ordinary skill in the art will appreciate that the sliders 514, 516 can be coupled to their respective jaws 511, 515 by other couplings. For example, by a biasing coupling, a spring coupling, or other coupling that allows for the axially translatable cooperation between the sliders 514, 516 and their respective jaws 511, 515. In at least one embodiment, at least one of the sliders 514, 516 can be biased towards the first position 2000, where and end 518, 519 of the slider 514, 516 is adjacent the second end 207 of the hollow elongate member 205. For example, the slider 514, 516 can be biased by a spring, a cam surface, or other mechanism configured to bias the slider 514, 516 toward the first position 2000. In another embodiment, only one of the sliders 514, 516 can be biased towards the first position.

In the second position 2500 of the trocar assembly 200, the first slider 514 and second slider 516 are able to translate along their respective jaw 511, 515. For example, in Figure 7, when an implement 400 is inserted and advanced through the hollow elongate member 205 such that the distal end 410 of the implement 400 engages an end 518, 519 of the first slider 514 and second slider 516. As the implement 400 is further advanced through the hollow elongate member 205 to protrude out from the second end 207 and to advance through the first jaw 511 and second jaw 515, the implement 400 can apply pressure against the first slider 514 and second slider 516 to translate the first slider 514 and second slider 516 away from the second end 207 of the hollow elongate member 205. For example, the first slider 514 and second slider 516 can translate axially away from the second end 207 of the hollow elongate member 205 as the first jaw 511 and second jaw 515 rotate radially away from the longitudinal axis 230 of the hollow elongate member 205 in the second position 2500 (for example, the expanded position). In other words, as the implement 400 is advanced through the hollow elongate member 205 and the first jaw 511 and second jaw 515, the implement 400 can assist in translating the first slider 514 and second slider 516 with respect to the first jaw 511 and second jaw 515. While Figure 7 illustrates a trocar assembly 200 having two sliders 514, 516, those of ordinary skill in the art will appreciate that the trocar assembly 200 can have one slider 514, 516.

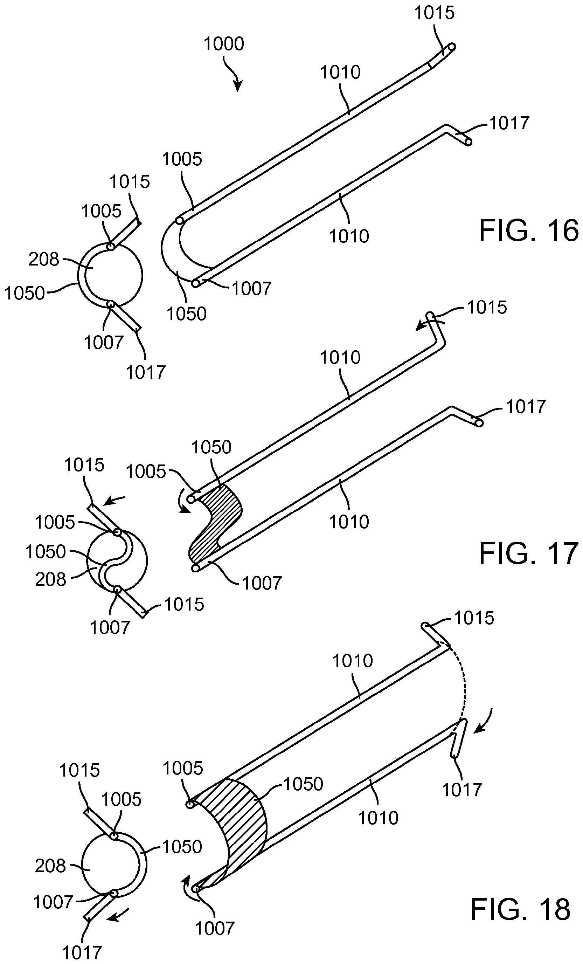

In the exemplary embodiment illustrated in Figure 7, the trocar assembly 200 can include a scope cleaner 700 interiorly positioned with respect to the jaws 510. For example, in Figure 7, the scope cleaner 700 can be a pair of wiper blades 705, 707 coupled to the jaws 510. The wiper blades 705, 707 can be rigid wiper blades, deformable wiper blades, tips of wiper blades, absorbent blades, or other structures which can wipe or sweep debris off of implements 400 insertable in the hollow elongate member 205. In Figure 7, a first wiper 705 can be coupled to the first jaw 511. In Figure 7, the first wiper blade 705 can be coupled to the first slider 514. In Figure 7, the first slider 714 has the penetrating member 513 coupled to a first end 512, and the first wiper blade 705 can be coupled to the first slider 514 at an end 518 opposite to the penetrating member 513. The first wiper blade 705 can be configured to sweep across an interior 208 (shown in Figures 16-18) of the hollow elongate member 205 when the first slider 514 translates axially away from the second end 207 of the hollow elongate member 205 and the first jaw 511 is rotated into the second position 2500. The second wiper blade 707 can be coupled to the second jaw 515. For example, in Figure 7, the second wiper blade 707 can be coupled to the second slider 516. In Figure 7, the second wiper blade 707 can be coupled to an end 519 of the second slider 516, such that in the first position 2000, the first wiper blade 705 and the second wiper blade 707 are adjacent one another. The second wiper blade 709 can be configured to sweep across an interior 208 (shown in Figures 16-18) of the hollow elongate member 205 when the second slider 516 translates axially away from the second end 207 of the hollow elongate member 205 and the second jaw 515 is rotated into the second position 2500. The sweeping movement of the first wiper blade 705 and second wiper blade 707 will be described in further detail with the exemplary non-limiting embodiment illustrated in Figure 9-13.

Figure 9 illustrates a cross-sectional view of the trocar assembly 200 illustrated in Figure 7 taken along the longitudinal axis 230 of the hollow elongate member 205. In Figure 9, the trocar assembly 200 is in the first position 2000 (e.g., rest position). A locking member 300, such as the one illustrated in Figure 8, is inserted in the hollow elongate member 205 and engaged with the first jaw 511 and second jaw 515 to maintain the trocar assembly 200 in the first position 2000. For example, as illustrated in Figure 9, the tabs 810 of the locking member 300 engage recesses 509 formed in an interior surface 507 of the first jaw 511 and second jaw 515. As the locking member engages recesses 509, the first jaw 511 an second jaw 515 are prevented from rotating axially away from the longitudinal axis 230 of the hollow elongate member 205. Also illustrated in Figure 9, as the locking member 300 maintains the trocar assembly 200 in the first position, the first wiper blade 705 and second wiper blade 705 are maintained adjacent one another and proximate to a center 206 of the cross-sectional face of the hollow elongate member 205. Figure 9 also illustrates a cross-sectional view of the trocar assembly 200 taken along a plane perpendicular to the first jaw 511 and second jaw 515 and the longitudinal axis 230. As illustrated in this cross-sectional view, when the locking member 300 maintains the trocar assembly 200 in the first position, the first wiper blade 705 and the second wiper blade 707 are maintained such that the first wiper blade 705 and second wiper blade 707 are adjacent to one another. In the event an implement 400 is inserted in the hollow elongate member 705 and the trocar assembly 200 is in the first position 2000, the first wiper blade 705 and the second wiper blade 707 can be adjacent to a distal end 410 of the implement 400. For example, as illustrated in Figure 9, the first wiper blade 705 and the second wiper blade 707 are disposed adjacent one another.

Figure 10 illustrates the trocar assembly 200 in Figure 9, except the locking member 300 has been removed from the hollow elongate member 205. As the locking member 300 has been removed, the trocar assembly 200 is permitted to transition from the first position 2000 (e.g., rest position) to the second position 2500. The trocar assembly 200 can be transitioned to the second position 2500 as the implement 400 is advanced through the hollow elongate member 205 to protrude out from the second end 207 of the hollow elongate member 205.

Figure 11 illustrates the trocar assembly 200 in Figures 9 and 10, where the implement 400 has been advanced towards the second end 207 of the hollow elongate member 205 such that the implement 400 begins to protrude out from the second end 207 and the first jaw 511 and second jaw 515 begin to rotate axially away from the longitudinal axis 230 of the hollow elongate member 205. That is, Figure 11 illustrates the trocar assembly 200 beginning to transition to the second position 2500 (e.g., expanded position). In Figure 11, as the implement 400 advances further through the hollow elongate member 205 to protrude out from the second end 207 of the hollow elongate member 205, the first slider 514 and second slider 516 translate axially away from the second end 207 of the hollow elongate member 205, thereby permitting the wiper blades 705, 707 to sweep across the exterior surface of the implement 400. Figure 11 also illustrates a cross-sectional view of the trocar assembly 200 taken along a plane parallel to the longitudinal axis 230. As illustrated in this cross-sectional view in Figure 11, as the implement 400 protrudes out from the second end 207 of the hollow elongate member 205 and engages the wiper blades 705, 707, the force of the implement 400 against the wiper blades 705, 707 can cause the wiper blades 705, 707 to sweep across the exterior surface of the implement 400. For example, as illustrated in Figure 11, the wiper blades 705, 707 can move radially away from the center 206 of the cross-sectional face of the implement 400 and the second end 207 of the hollow elongate member 205. That is, the wiper blades 705, 707 can sweep outwardly across the face of the implement 400 beginning from the center 206 of the implement 400 towards the circumference of the implement 400.

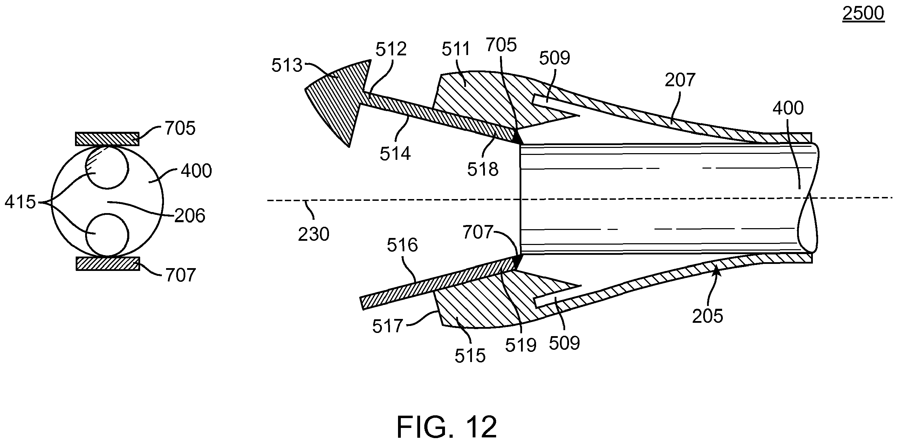

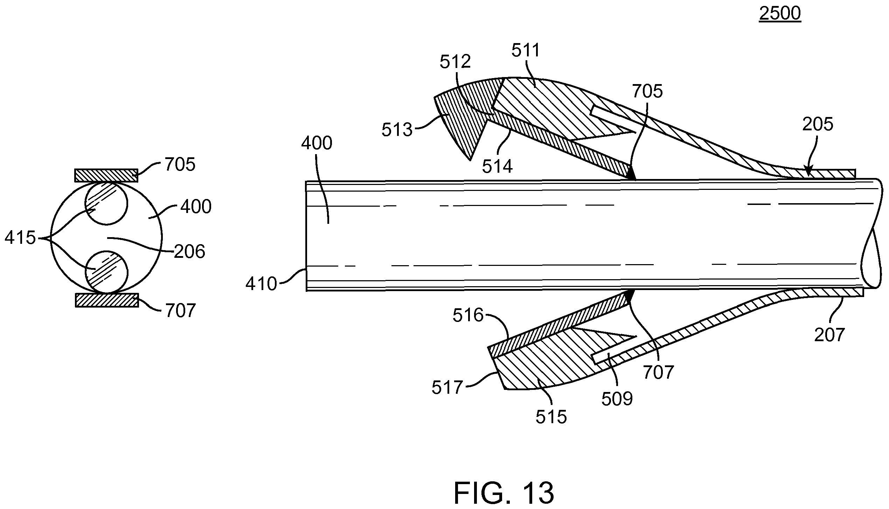

Figure 12 illustrates the trocar assembly 200 in the second position 2500 (e.g., expanded position). In the non-limiting exemplary embodiment illustrated in Figure 12, the implement 400 has been advanced through the hollow elongate member 205 beyond the second end 207 of the hollow elongate member 205. As illustrated in Figure 12, the implement 400 has been advanced beyond the second end 207 of the hollow elongate member 205 such that the implement 400 can advance between the first jaw 511 and second jaw 515. As illustrated in Figure 12, as the implement 400 is further advanced through the hollow elongate member 205 and between the first jaw 511 and second jaw 515, the wiper blades 705, 707 sweep further outwardly across the exterior face of the implement 400 until the wiper blades 705, 707 are positioned proximate the circumference of the implement. If the implement 400 is advanced even further through the hollow elongate member 205 and between the first jaw 511 and the second jaw 515, the implement 400 can be advanced to protrude beyond the second end 517 of the second jaw 515 and beyond the penetrating member 213 of the first jaw 511, as illustrated in Figure 13.

As illustrated in Figure 13, when the implement 400 is advanced such that it protrudes beyond the second end 517 of the second jaw 515 and the penetrating member 513 of the first jaw 511, the wiper blades 705, 707 can remain positioned adjacent the circumference of the implement 400. In at least one non-limiting exemplary embodiment, as illustrated in Figure 13, the first slider 514 and second slider 516 can be biased towards the second end 207 of the hollow elongate member 205. For example, the first slider 514 and the second slider 516 can be biased such that the first slider 514 and second slider 516 form a contiguous contour with their respective first jaw 511 and second jaw 515, as illustrated in Figure 13. For example, each of the first slider 514 and the second slider 516 can be biased towards the second end 207 of the hollow elongate member by a biaser such as a spring, a cam surface, or other biasing member.

In Figures 9-13, as the wiper blades 705, 707 can be swept across the exterior of the implement 400, the wiper blades 705, 707 can wipe debris from the implement 400. For example, if the implement 400 is an endoscope, as illustrated in Figure 9-13, the sweeping action of the wiper blades 705, 707 can wipe debris from the endoscope, thereby increasing the surgeon's visibility of the body cavity in which the endoscope is inserted to perform surgical procedures. The wiper blades 705, 707 can also reduce "fogging" of the lens or clean the lens of the endoscope. With the embodiment of the scope cleaner 700 illustrated in Figures 7 and 9-14, a surgeon need not remove the endoscope 400 from the body cavity to clean the endoscope 400. That is, the surgeon need only retract and advance the endoscope 400 from the trocar assembly 200. For example, in Figure 13, as the endoscope 400 protrudes beyond the second end 517 of the second jaw 515 and the penetrating member 513 of the first jaw 511, the endoscope 400 can be manipulated by the surgeon during medical procedures. During these medical procedures, the endoscope 400 can accumulate debris thereon. In the event the surgeon's visibility from the endoscope 400 becomes obstructed, the endoscope 400 can be retracted within the hollow elongate member 205 such that the trocar assembly 200 is in the first position 2000 (e.g., the rest position). The endoscope 400 can then be advanced through the hollow elongate member 205 and out beyond the second end 517 of the second jaw 515 and the penetrating member 513 of the first jaw 511, thereby permitting the wiper blades 705, 707 to sweep across the exterior surface of the endoscope and to wipe debris off of the endoscope 400. As a result, the surgeon's visibility from the endoscope 400 is clearer as the endoscope 400 is cleaned by the wipers 705, 707. While Figure 9-13 illustrate the wiper blades 705, 707 having a length that is longer than the diameter of the lenses 415, 415', those of ordinary skill in the art will appreciate that the wiper blades 705, 707 can have a length that is substantially equal to the entire diameter of the lenses 415, 415', a length that is substantially equal to an inner diameter of the hollow elongate member 205, a length that is 80 percent of the inner diameter of the hollow elongate member 205, or any other length which can clean debris off of the endoscope 400 or implement inserted into the trocar assembly.

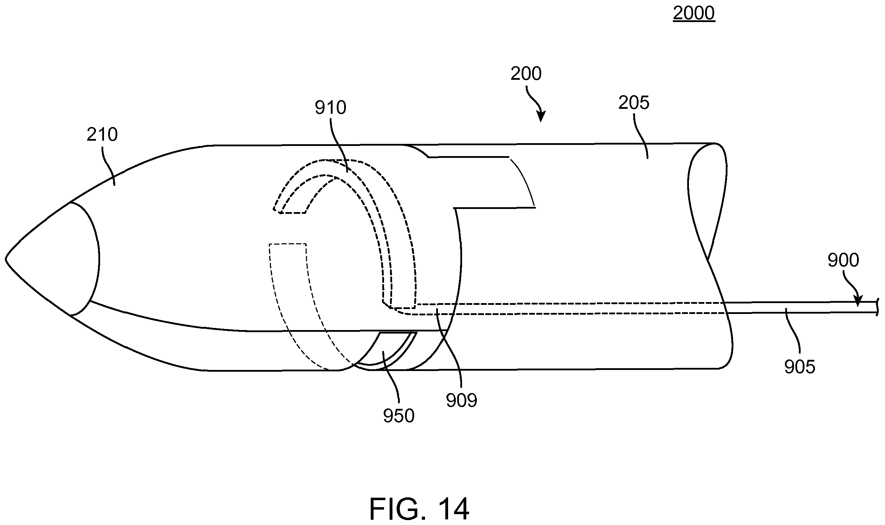

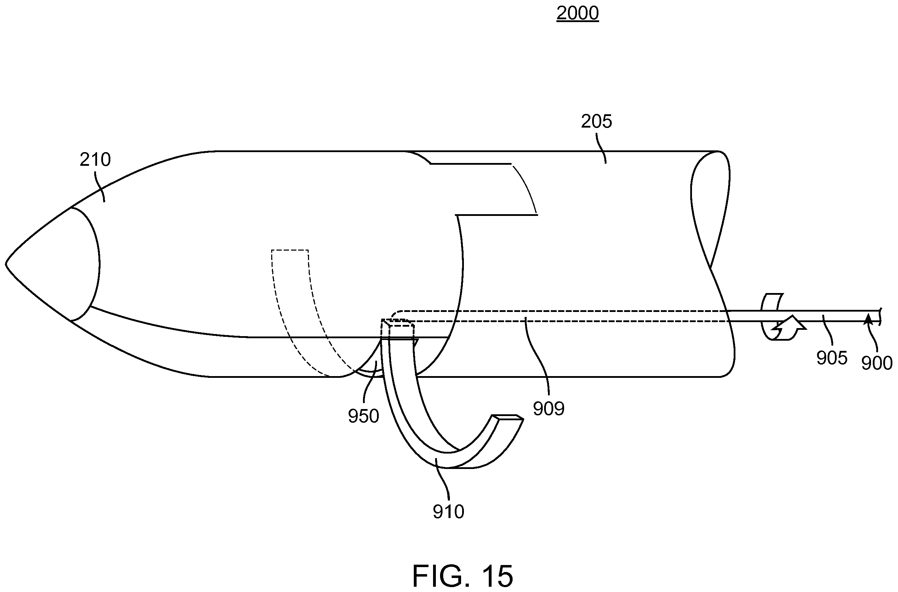

Figures 14 and 15 are an illustration of another non-limiting exemplary embodiment of a trocar assembly 200 of the present disclosure having an alternative embodiment of a scope cleaner 900. In Figures 14 and 15, the scope cleaner is a rotatable scope cleaner 900. For example, the rotatable scope cleaner 900 comprises a rotatable stem 905 and a wiper blade 910. The rotatable stem 905 can be actuated by the surgeon. The wiper blade 910 is rotatably coupled to a distal end 909 of the stem 905. For example, a rotation of the stem 905 causes the wiper blade 910 to rotate. As the wiper blade 910 rotates, the wiper blade 905 can sweep across the cross-sectional face of an implement 400 inserted into the hollow elongate member 205 and/or an interior of the hollow elongate member 205. In Figures 14 and 15, the wiper blade 910 is a rotatable arched blade. For example, in Figures 14 and 15, the at least one jaw 210 includes an aperture 950 formed therein. The aperture 950 is configured such that the rotatable wiper blade 910 can pass therethrough when the wiper blade 910 is rotated. Figure 14 illustrates a starting position of the rotatable blade 910 where the rotatable blade 910 is positioned parallel to an interior circumference of the at least one jaw 210. When the endoscope 400 is inserted and/or retracted into the hollow elongate member 205, and the trocar assembly 200 is placed in the first position 2000 (e.g., rest position), the endoscope 400 can be positioned adjacent the rotatable wiper blade 910 such that the wiper blade 910 engages the exterior surface of the endoscope 400. The rotatable stem 905 can be actuated to rotate the wiper blade 910. For example, the wiper blade 910 can be rotated outwardly through the aperture 950, such that the wiper blade 910 is positioned externally to the at least one jaw 210. Figure 15 illustrates an end position of the rotatable blade 910 after the rotatable stem 910 has been rotated to rotate the wiper blade 910 outwardly through the aperture 950. As the wiper blade 910 is rotated outwardly through the aperture 950, the wiper blade 910 can sweep across the exterior surface of the endoscope 400, thereby cleaning the endoscope and removing debris therefrom. In Figures 14 and 15, the rotatable wiper blade 910 can be arched to maximize the surface area of the endoscope cleaned or wiped by the wiper blade. However, in other embodiments, the wiper blade 910 can have other shapes and configurations, so long as the wiper blade 910 can be manipulated to clear debris from the surface of the endoscope, thereby increasing or clearing the surgeon's visibility of the endoscope 400.

Figures 16-18 illustrate an alternative embodiment of a scope cleaner 1000. In Figures 16-18, the scope cleaner is a rotatable flexible wiper blade assembly 1000. In Figures 16-18, the wiper blade assembly 1000 can include a flexible wiper blade 1050 coupled to a lever 1015, 1017. The flexible wiper 1050 can be configured to be positioned between the jaw (not shown) and the second end (not shown) of a hollow elongate member (not shown) to which the flexible wiper 1050 can be coupled. In Figures 16-18, the flexible wiper blade 1050 can be coupled to two levers 1015, 1017. A rotation of the levers 1015, 1017 can cause the flexible wiper 2050 to sweep across an interior 208 of the hollow elongate member 205 in which the flexible wiper blade assembly 1000 is inserted. However, those of ordinary skill in the art will appreciate that the wiper blade 1050 can be coupled to only one lever.

In Figures 16-18, a first end 1005 of the wiper blade 1050 can be coupled to the first lever 1015. For example, in Figures 16-18, the first end 1005 can be coupled to the first lever 1015 by a stem 1010 configured to extend longitudinally parallel to the hollow elongate member 205 in which the flexible wiper blade assembly 1000 is inserted. The second end 1007 of the flexible wiper blade 1050 can be coupled to the second lever 1017. In at least one embodiment, such as in Figures 16-18, the second end 1007 of the flexible wiper blade 100 can be coupled to the second lever 1017 by a respective stem 1010 configured to extend longitudinally parallel to the hollow elongate member 205. As illustrated in Figures 16-18, the flexible wiper blade 1050 can extend along a diameter of the interior of a hollow elongate tube in which the flexible wiper blade assembly 1000 is inserted. In Figures 16-18, the flexible wiper blade 1050 extends across an entirety of the diameters to the hollow elongate member to ensure that an entirety of the cross-sectional surface of the hollow elongate member will be cleaned. However, in other embodiments, the flexible wiper blade 1050 can extend partially across the diameter of the hollow elongate member or can extend across the interior of the hollow elongate member in any other manner that allows the wiper blade 1050 to sweep across at least a portion of the interior of the hollow elongate member.

In Figures 16-18, a rotation of the levers 1015, 1017 can cause the flexible wiper 2050 to sweep across an interior 208 of the hollow elongate member 205 in which the flexible wiper blade assembly 1000 is inserted. For example, in Figure 17, the first lever 1015 has been rotated counterclockwise. In response to this counterclockwise rotation of the first lever 1015, the first end 1005 of the flexible wiper blade 1050 is also rotated counterclockwise. As the second end 1007 of the flexible wiper blade 1050 has not been rotated, the flexible wiper blade 1050 deforms or flexes. For example, as illustrated in Figure 17, the flexible wiper blade 1050 can flex and form a reverse-S-shape. As the flexible wiper blade 1050 can flex, the flexible wiper blade 1050 can sweep across approximately half of an interior 208 of the hollow elongate member 205 in which the flexible wiper blade assembly 1000 is inserted. In another embodiment, the flexible wiper blade 1050 can sweep across an exterior surface of an implement (not shown), for example, across the lens of an endoscope. In the exemplary embodiment illustrated in Figures 16-18, the flexible wiper blade 1050 can be flexed to sweep across the remaining half of the interior 208 of the hollow elongate member 205 upon a rotation of the second lever 1017. For example, in Figure 18, the second lever 1017 can be rotated clockwise, which thereby rotates the second end 1007 of the flexible wiper blade 1050 to also rotate counterclockwise. As a result of this rotation, the flexible wiper blade 1050 can continue to flex. As the rotation of the first lever 1015 can cause the wiper blade 1050 to flex into a reverse-S-shape, the rotation of the second lever 1017 can cause the wiper blade 1050 to continue to flex out of the reverse-S-shape. For example, the rotation of the second lever 1017 can cause the flexible wiper blade 1050 to flex into a semi-circular shape, as illustrated in Figure 18. In Figure 18, the flexible wiper blade 1050 has flexed to a position that is a mirror image of the beginning position of the flexible wiper blade 1050 before either of the levers 1015, 1017 has been rotated. As illustrated in Figure 18, the flexible wiper blade 1050 has swept across substantially an entirety of the interior 208 of the hollow elongate member 205.

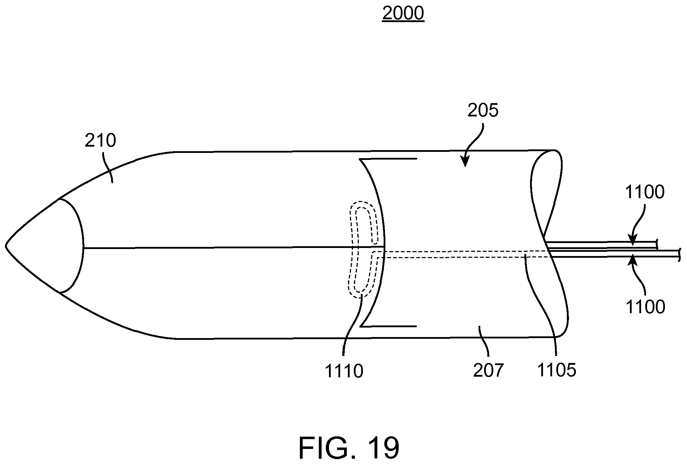

Figures 19-21 illustrate an exemplary non-limiting embodiment of a trocar assembly 200 having a biasing mechanism 1100. The biasing mechanism 1100 can be configured to maintain the trocar assembly 200 in the second position 2500. For example, the biasing mechanism 1100 can maintain the at least one jaw 210 of the trocar assembly 200 in a position where the end 213 of the jaw is positioned radially away from the longitudinal axis (for example, a longitudinal center line) of the hollow elongate member 205. By biasing the trocar assembly 200 in the second position 2500 (e.g., the expanded position), implements 400 (not shown) can be inserted and removed during medical procedures performed within the body cavity without having to continually transition the at least one jaw 210 between the first position 2000 and the second position 2500. As the trocar assembly 200 can be biased in the second position 2500 after being inserted into the body cavity of the patient, there can be fewer moving parts within the body cavity and less irritation to the interior of the body cavity.

Those of ordinary skill in the art will appreciate that the scope cleaners 700, 900, 1000 illustrated in Figures 6-16 can be removably coupled to the trocar assembly 200. For example, with the scope cleaner 700 illustrated in Figures 9-13, the scope cleaner 700 can be detachable from the jaw 210. In other embodiments, the scope cleaners can be removably insertable from the hollow elongate member 205.

While Figure 6-16 illustrate scope cleaners 700, 900, 1000 that include a wiping blade mechanism, those of ordinary skill in the art will appreciate that other scope cleaners can be implemented. For example, the scope cleaner can include a cleaning spray 590 (for example, as illustrated in Figure 5). The cleaning spray 590 can include a fluid line 591, 592 connected to an actuator (not shown), which when actuated sprays fluid from the fluid line 591, 592. The fluid can be a liquid or a gas. The fluid can be a cleaning fluid or a drying fluid. In Figure 5, the cleaning spray includes two fluid lines 591, 592. However, those of ordinary skill in the art will appreciate that one fluid line can be used or more than two fluid lines can be used. In Figure 5, one of the fluid lines 591 can spray a cleaning fluid onto a lens of an endoscope inserted in the trocar assembly 200. The other fluid line 592 can spray a drying fluid onto the lens of the endoscope.

Those of ordinary skill in the art will also appreciate that the scope cleaner can be offset laterally or rotated on a hinge offset from the at least one jaw 210 to position the scope cleaner away from the path of an implement 400 inserted into the trocar assembly 200.

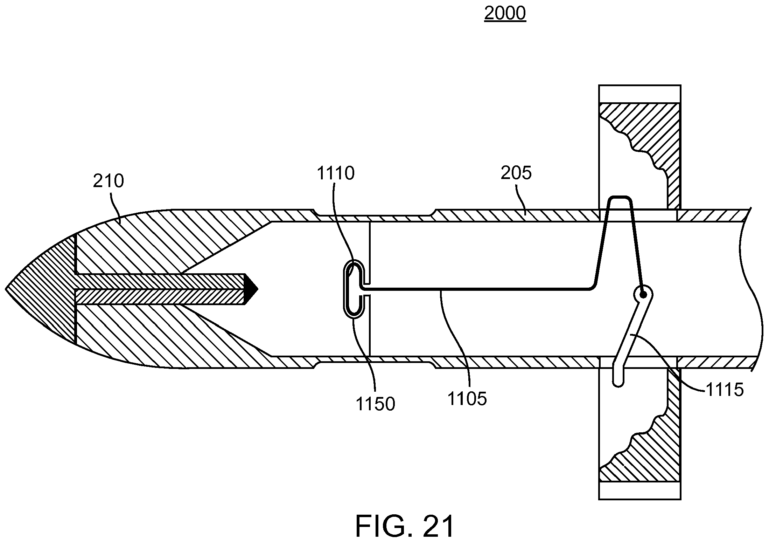

In Figures 19-21 the biasing mechanism 1100 includes a rod 1105 and a bias loop 1110. The bias loop 1110 can be a rigid wire shaped to conform to a recess 1150 (shown in Figure 21) formed within a wall of the at least one jaw 210the at least one jaw 210 of the trocar assembly 200. In Figures 19-21, the bias loop 1110 has an oblong shape; however, those of ordinary skill in the art will appreciate that the bias loop 1110 can have other shapes. Some examples of other shapes include a wedge like shape or a round disk like shape. In Figures 19-21, the bias loop 1110 has a first end configured to engage a first recess of the at least one jaw 210the at least one jaw 210. The bias loop 1110 can also have a second end configured to engage a second recess of the at least one jaw 210. As illustrated in Figure 21, the recess 1150 can be formed in an outer wall of the at least one jaw 210. The recess 1150 can have a rounded shape to accommodate a rounded portion of the bias loop 1110. However, those of ordinary skill in the art will appreciate that the recess 1150 can have any other shape, so long as the recess 1150 can accommodate the bias loop 1110.

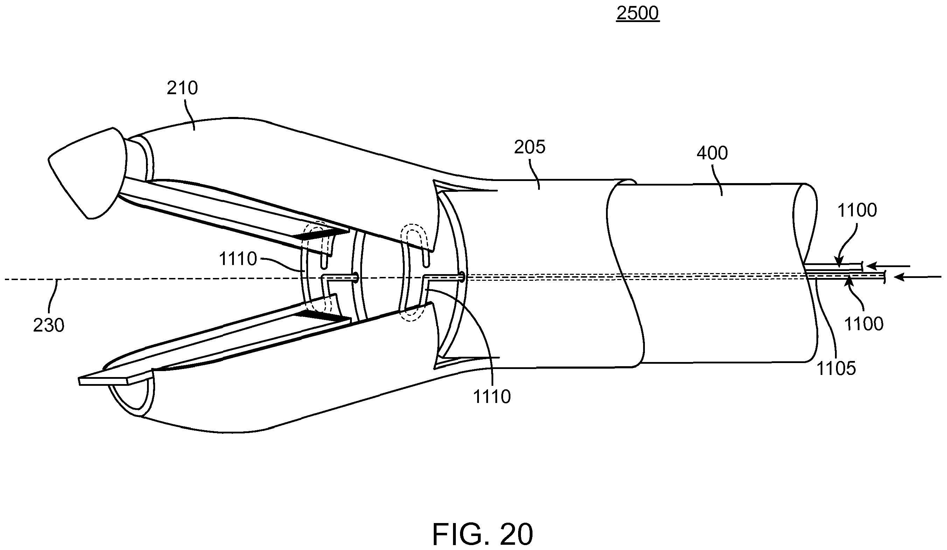

In Figures 19-21, the bias loop 1110 can have pressure applied thereto to urge the at least one jaw 210 of the trocar assembly 200 towards the second position 2500 and maintain the trocar assembly 200 there. For example, as illustrated in Figure 21, the bias loop 1110 can be coupled to an actuator 1115. For example, the actuator 1115 can be a lever, a knob, a dial, or any other actuator which can be actuated to place pressure onto the bias loop 1110. In Figure 21, the bias loop 1110 is coupled to the actuator 1115 by a substantially rigid wire; however, in other embodiments, the bias loop 1110 can be coupled to the actuator by a rod, a stem, or other member which can transfer pressure from the actuator 1115 to the bias loop 1110. As illustrated in Figures 19-20, when the actuator 1115 is actuator, pressure is placed on the bias loop 1110, which thereby places pressure against the interior of the recess 1150 of the jaw. As pressure is placed against the interior of the recess 1150, the at least one jaw 210 can be rotated from the first position 2000, illustrated in Figure 19, to the second position 2500, illustrated in Figure 20. When the actuator 1115 is maintained in a position that constantly applies pressure to the bias loop 1110, the trocar assembly 200 can be maintained in the second position 2500 (e.g., expanded position). When the actuator 1115 is released from the position that applies pressure to the bias loop 1110, pressure is removed from the bias loop 1110, and the at least one jaw 210 can be transitioned back to the first position 2000 (e.g., rest position) illustrated in Figure 19. Figures 19-21 illustrate a biasing mechanism 1100 that includes two biasing loops 1110; however, those of ordinary skill in the art will appreciate that the trocar assembly 200 can have fewer or more than two loops 1110. Additionally, while Figures 19-21 illustrate a biasing mechanism 1100 that includes an actuator 1115 and a biasing loop 1100, those of ordinary skill in the art will appreciate that other biasing mechanisms 1110 can be utilized to maintain the trocar assembly 200 in the second position 2500. For example, the biasing mechanism 1100 can be a plug, a stopper, or other mechanism which can bias the trocar assembly in the second position 2500.

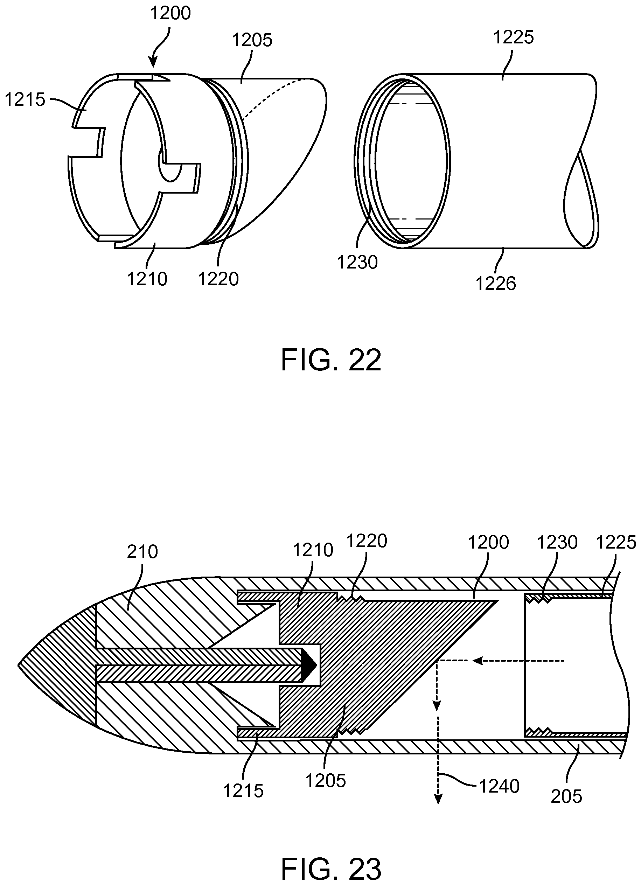

In at least one embodiment, the jawed trocar assembly 200 can include a reflecting member 1200, as illustrated in Figures 22 and 23. The reflecting member 1200 can allow for a radial view by an endoscope (not shown) inserted therein. For example, an endoscope can be inserted into the jawed trocar assembly to provide an axial view of the body cavity into which the jawed trocar assembly 200 is inserted. However, in some instances, the optics looking axially can be difficult due to shallow angles and reflections. A reflecting member 1200, for example, as illustrated in Figure 22, can enhance the views provided by the endoscope. For example, the reflecting member 1200 can have a reflective surface which can reflect an image of the body cavity or the incision surface of the body tissue to be penetrated to an endoscope inserted into the jawed trocar assembly. That is, the body cavity or body tissue to be penetrated which is radially adjacent to the jawed trocar assembly 200 when the jawed trocar assembly 200 is inserted in the body cavity can be reflected via the reflecting member 1200 to the endoscope inserted in the hollow elongate member 205 of the jawed trocar assembly 200.

Figure 22 illustrates an exemplary embodiment of such a reflecting member 1200. In Figure 22, the reflecting member 1200 can be a cylindrical member sized to fit within the hollow elongate member 205 of the jawed trocar assembly 200. The reflecting member 1200 can have a reflective portion 1205 which can reflect an image of the area radially adjacent to the trocar assembly 200 to an endoscope inserted in the hollow elongate member 205. For example, the reflective portion 1205 can be a mirror. As illustrated in Figure 22, the reflective portion 1205 can have an angled surface (not labeled) to further enhance the radial view of the endoscope. For example, the angled surface can be angled: forty-five degrees along the diameter of the reflective portion 1205 (as illustrated in Figure 22), sixty-degrees along the diameter of the reflective portion 1205, forty-degrees along the diameter of the reflective portion 1205, forty-five degrees along a radius of the reflective portion 1205, forty-five degrees along a portion of the diameter of the reflective portion 1205, or any other along the reflective portion 1205 so long as areas radially adjacent to the trocar assembly 200 can be reflected axially into the hollow elongate member 205 to an endoscope inserted therein. As the angled surface of the reflective portion 1205 can reflect images of areas radially adjacent to the trocar assembly to an endoscope axially inserted therein, information about the penetration and breakthrough areas of the body cavity and body tissue can be readily viewed by the operator of the endoscope.

The reflecting member 1200 can have a locking portion 1210 coupled thereto, as illustrated in Figures 22 and 23. In Figure 22, the locking portion 1210 can be a hollow cylindrical member formed on a side of the reflecting member 1200 opposite to the reflective portion 1205. The locking portion 1210 can be configured to lock the jaws 210 of the trocar assembly 200 in the first position 2000 (for example, the resting position). The locking portion 1210 can operate similar to the locking member 300 discussed above. In Figures 22 and 23, at least one locking tab 1215 is formed on the locking portion 1210. For example, in Figure 22, four locking tabs 1215 are formed on a circumference of the locking portion 1210. However, those of ordinary skill in the art will appreciate that there can be fewer or more locking tabs 1215 than as illustrated in Figure 22. Those of skill in the art will also appreciate that the locking tabs 1215 can be locking feet, locking posts, locking pegs, or other locking members configured to engage a recess of the trocar assembly 200 to maintain the at least one jaw 210 in the rest position 2000.

In Figures 22 and 23, the reflecting member 1200 can include a placement tool 1225. The placement tool 1225 can be releasably couplable to the reflecting member 1200 to insert the reflecting member 1200 in the hollow elongate member 205 of the trocar assembly. In Figures 22 and 23, the placement tool 1225 can be a hollow member 1226 in which the reflecting member 1200 can be received. For example, in Figure 22, the reflective portion 1205 can include a threaded portion 1220 configured for mating engagement with a respective threaded portion 1230 formed on the placement tool 1225. Those of ordinary skill in the art will appreciate that the reflective portion 1205 can be releasably couplable to the placement tool 1225 by other mechanism such as a snap-fitting, a conformance fitting, a twist-and-release fitting, or other releasable coupling.

When the reflecting member 1200 is to be inserted into the trocar assembly 200, the reflecting member 1200 can be coupled to the placement tool 1225 prior to inserting the reflecting member 1200 into the trocar assembly. For example, the threaded portion 1220 of the reflective portion 1205 of the reflecting member 1200 can be matingly threaded to the threaded portion 1230 of the placement tool 1225, thereby securing the reflecting member 1200 to the placement tool 1225. The placement tool 1225 can then be inserted into the hollow elongate member 205 of the trocar assembly 200 and advanced therethrough until the locking portion 1215 of the reflecting member 1200 engages the at least one jaw 210 of the trocar assembly 200 to lock the trocar assembly 200 in the rest position 2000. The placement tool 1225 can disengage the reflective portion 1205. For example, as illustrated in Figure 23, the placement tool 1225 can disengage the reflective portion 1205 by rotating the placement tool 1225 in a direction that unmates the threaded portion 1230 of the placement tool 1225 from the threaded portion 1220 of the reflecting member 1200. When the placement tool 1225 is disengaged from the reflecting member 1200, the placement tool 1225 can be removed from the hollow elongate member 205 of the trocar assembly 200. The reflecting member 1200 can remain inside the hollow elongate member 205. In Figure 23, the hollow elongate member 205 can have optically semi-transparent walls. For example, the hollow elongate member 205 can be clear, transparent, semi-transparent, or otherwise see-through so that images of areas adjacent to the hollow elongate member 205 can be reflected by the reflective surface 1205 of the reflecting member 1200. In Figure 23, in the event an endoscope or other camera implement is inserted in the hollow elongate member, the reflective portion 1205 can reflect images of areas adjacent to the hollow elongate member 205 to the endoscope or camera implement. For example, as shown in Figure 23, the reflected images can follow the radial viewing path 1240.When the trocar assembly 200 is to be transitioned into the expanded position (for example, as illustrated in Figure 20), the placement tool 1225 can be inserted into the hollow elongate member 205, can matingly engage the reflecting member 1200, and can be removed from the hollow elongate member 205, thereby permitting the at least one jaw 210 of the trocar assembly 200 to transition into the expanded position.

The reflecting member 1200 thereby enables the camera implement to transmit an image reflected by the reflecting member 1200 to the operator of the camera implement. Thus, with the reflecting member 1200 and the camera implement, the operator of the trocar assembly can receive: enhanced views of the body tissue to be penetrated by the trocar assembly 200 and side views and radial views of the interior of the body cavity when the trocar assembly 200 is inserted therein.

While the Figures 1-23 illustrate a jawed trocar assembly in which the at least one jaw 210 is formed on or permanently coupled to the hollow elongate member 205, those of ordinary skill in the art will appreciate that the at least one jaw 210 can be releasably coupled to the hollow elongate member 205. For example, the at least one jaw 210 and the end of the hollow elongate member 205 to which the at least one jaw 210 can have corresponding threaded engagement portions. That is, the at least one jaw 210 can be releasably coupled to the hollow elongate member 205 by screwing the at least one jaw 210 to the hollow elongate member 205. In other embodiments, the at least one jaw can 210 can be releasably coupled to the hollow elongate member 205 by a snap-fit engagement or any other releasable coupling. By having a releasably couplable at least one jaw 210, the jaw 210 can be disposable or reusable. The jaw 210 can also be configured to fit existing trocar members, disposable trocars, or other hollow elongate member. In another embodiment, the hollow elongate member 205 can have a slit extending parallel to the longitudinal axis of the hollow elongate member 205 and extending along a majority of the longitudinal axis. For example, the slit can extend along 30 percent, 50 percent, 75 percent, or any other majority of the longitudinal axis.

While Figures 1-23 illustrate a pair of jaws having symmetric jaws, those of ordinary skill in the art will appreciate that the jaws need not be symmetrical. For example, the jaws can be asymmetrical with respect to each other. That is, one jaw can have a greater surface area than the other, one jaw can be larger in size as compared to the other, one jaw can have a contour different from the other jaw, or one jaw can be otherwise different from the other jaw.

A method of inserting an endoscopic tool assembly into a body cavity, where the endoscopic tool assembly includes any one of the trocar assemblies 200 described herein and illustrated herein, can include making an incision through a first body tissue. The trocar assembly can be inserted into the incision. The trocar assembly 200 can be advanced through the incision such that the at least one jaw 210 of the trocar assembly 200 engages a second body tissue. The trocar assembly 200 can be urged against the second body tissue to penetrate through the second body tissue. The endoscopic tool can be inserted through the trocar assembly 200. The endoscopic tool can be advanced through the trocar assembly 200 beyond the second end 207 of the elongate hollow member 205, thereby transitioning the trocar assembly 200 from the rest position 2000 to the expanded position 2500. The endoscopic tool can be extended beyond the end 213 of the at least one jaw 210.

In at least one embodiment, a locking member 300 can be inserted through the trocar assembly 200 prior to inserting the trocar assembly 200 through the incision. This can thereby ensure that the at least one jaw 210 is rigid enough to penetrate through the second layer of body tissue. The locking member 300 can be removed from the trocar assembly 200 after the trocar assembly 200 has been advanced to penetrate the second body tissue, thereby allowing for the insertion of the endoscopic tool or any other implement 400 to be used for medical procedures within the body cavity.

A method of cleaning an endoscopic tool assembly inserted into a body cavity, where the endoscopic tool assembly includes any one of the trocar assemblies 200 having a scope cleaner described and illustrated herein, can include urging the trocar assembly 200 against a body tissue to penetrate through the body tissue. An endoscopic camera of the endoscopic tool can be inserted though the trocar assembly 200. The endoscopic camera can be advanced through the trocar assembly 200 beyond the second end of the elongate hollow member, thereby transitioning the trocar assembly from the rest position 2000 to the expanded position 2500. The endoscopic camera can be extended beyond the end 213 of the at least one jaw 210 to expose the lens 415 of the endoscopic camera 400 to the body cavity. In the event the lens 415 of the camera becomes dirty or accumulates debris that impedes the visibility of the surgeon during the medial procedure, the endoscopic camera 400 can be retracted into the elongate hollow elongate member 205 of the trocar assembly 200. This can thereby transition the trocar assembly 200 from the expanded position 2500 to the rest position 2000. The lens 415 can engage with the scope cleaner of the trocar assembly 200 thereby cleaning debris off of the lens 415. The endoscopic camera 400 can be reciprocated within the elongate hollow member 205 such that the lens 415 is reciprocated between being exterior to the end 213 of the at least one jaw 210 and being interior to the at least one jaw 210 and engaged with the scope cleaner.

In another embodiment, the endoscopic camera 400 can be rotated within the hollow elongate member 205 after the endoscopic camera 400 has been retracted into the hollow elongate member 205 and after the lens 415 has been engaged with the scope cleaner. The rotations of the endoscopic camera 400 can cause the scope cleaner to wipe debris off of the lens 415.

In yet another embodiment, the endoscopic camera 400 can be cleaned by actuating a lever of the scope cleaner after the lens 415 has been engaged with the scope cleaner. The actuation of the lever can cause a wiper of the scope cleaner to undulate across the surface of the lens to wipe debris of the lens. In another exemplary embodiment, actuating a lever of the scope cleaner after the lens has been engaged with the scope cleaner can cause a spray of a cleaning fluid against the lens. For example, the cleaning fluid can be a liquid, a gas, or can include both liquid and gas. For example, the scope cleaner can include two lines of cleaning fluid positioned interiorly adjacent to the end of the jaw, and actuating the lever can spray the cleaning fluid from each of the two lines. However, those of skill in the art will realize that there may be fewer or more lines of cleaning fluid for cleaning a lens.

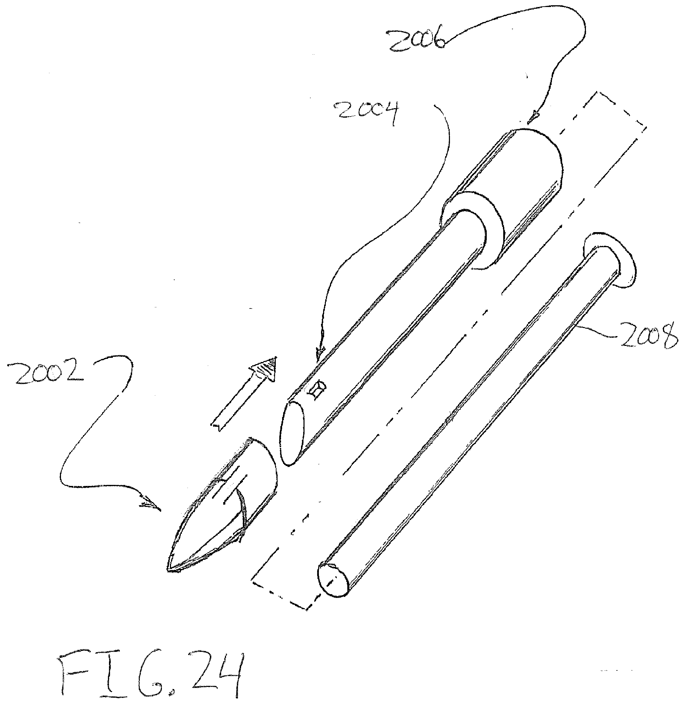

As illustrated in Figure 24, the trocar assembly can take the form of a removable trocar tip assembly 2002. The removable trocar tip assembly 2002, for example a removable trocar cap, can be configured to be mated with a trocar 2006 which does not include the features as described herein. The trocar tip assembly 2002 allows for a trocar to be assembled and manufactured so that the trocar assembly 2002 can be optionally included. This results in reduced tooling, while allowing increased selection and performance by the operator. The trocar tip assembly 2002 can be sized so as to mate with a desired trocar 2006. Additionally, the trocar 2006 can include a coupling device 2004. The coupling device 2004 allows for the trocar tip assembly 2002 to be coupled to the trocar 2006 in a fashion so as to resist removal. As illustrated the trocar cap coupling device 2004 is at least one catch pocket formed on the trocar 2006. The coupling device 2004 can be other types of mechanisms to assist in the retention of the trocar tip assembly 2002 once it is installed on the trocar 2006, but allow removal of the trocar tip assembly 2002 under certain circumstances. Additionally the trocar tip assembly 2002 can include a corresponding coupling device (not shown). The trocar tip assembly 2002 can include at least a lens wiper as described herein to clean the lens of a camera. Additionally, an instrument sleeve 2008 is included which allows for operation of the trocar cap 2002, which includes at least one of the features as described herein. For example, the instrument sleeve 2008 can slip inside of the trocar 2006. The instrument sleeve then can be used to open the trocar tip assembly 2002.

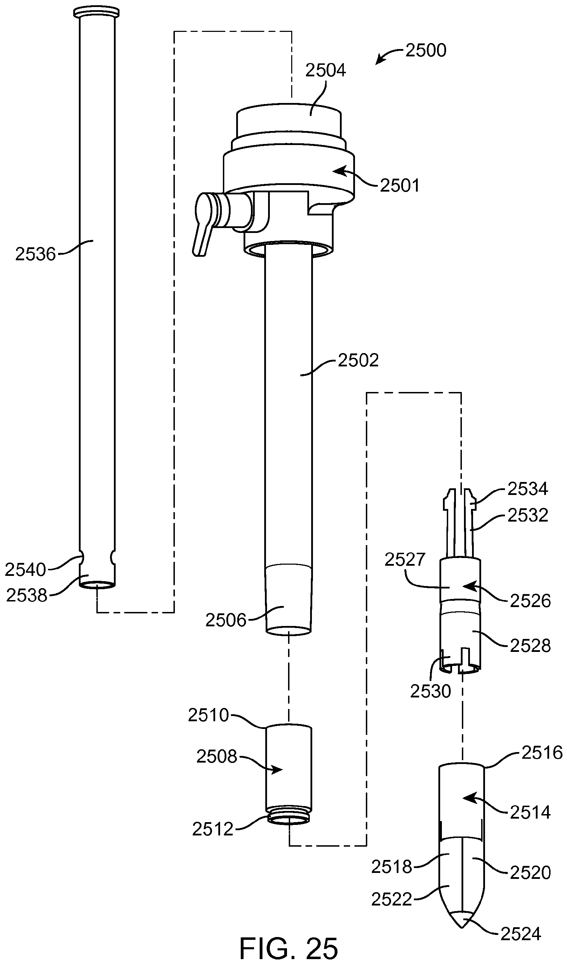

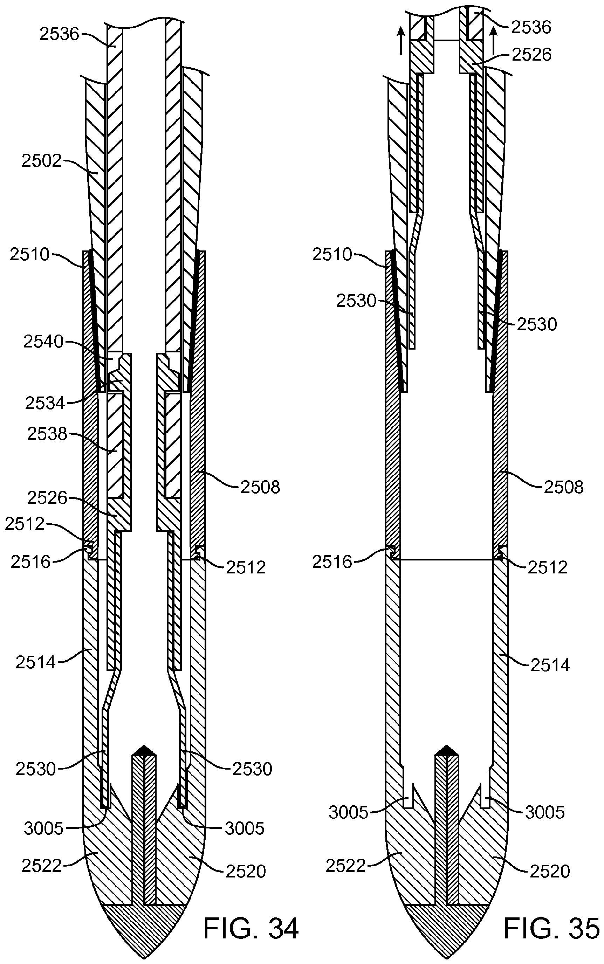

In at least one embodiment, the trocar assembly can include a trocar and a tip assembly removably couplable to the trocar as illustrated in Figures 25-35. The tip assembly 2508, 2514 allows for retrofitting existing trocars to turn a trocar which does not include the features as described in Figures 1-23 into one that has a jawed trocar tip, such as the one illustrated in Figure 24, for example. As will be described with respect to Figures 25-35, the tip assembly 2508, 2514 is couplable to an end of an existing or "off the shelf" trocar such that the existing trocar is provided with a jawed assembly at the end thereof.

Figure 25 is an exploded view of a trocar assembly 2500 including a trocar 2501, a tip assembly 2508, 2514, a locking member 2526 (such as the locking members discussed above), and a locking-member-removal tool 2536. The trocar 2501 illustrated in Figure 25 is an existing trocar. For example, a previously-purchased trocar, an off-the shell trocar, a commercially available trocar, a third-party trocar (such as a trocar manufactured or sold by a party other than the manufacturer or vendor of the tip assembly 2508, 2514), a separately-sold trocar, or any other existing trocar. The existing trocar 2501 can include an elongate member 2502 having a first end 2504 and a second end 2506. The first end 2504 can be a proximal end, such as the end that will be closest to the hand of the operator. The first end 2504 can be the end through which endoscopic implements or other implements are inserted during endoscopic procedures. The second end 2506 can be a distal end, such as the end that will be inserted into body cavity during endoscopic procedures.

A tip assembly 2508, 2514 can be coupled to the second end 2506 of the trocar 2501. The tip assembly 2508, 2514 can include adapter sleeve 2508 and the jaw assembly component 2514. As illustrated in Figure 25, the adapter sleeve 2508 can be a hollow cylindrical sleeve configured to receive the second end 2506 of the trocar 2501. While Figure 25 illustrates the adapter sleeve 2508 as a hollow cylindrical sleeve, those of ordinary skill in the art will appreciate that the adapter sleeve can be a hollow structure of any other shape that is configured to couple the jaw assembly component 2514 to the second end 2506 of the trocar 2501.

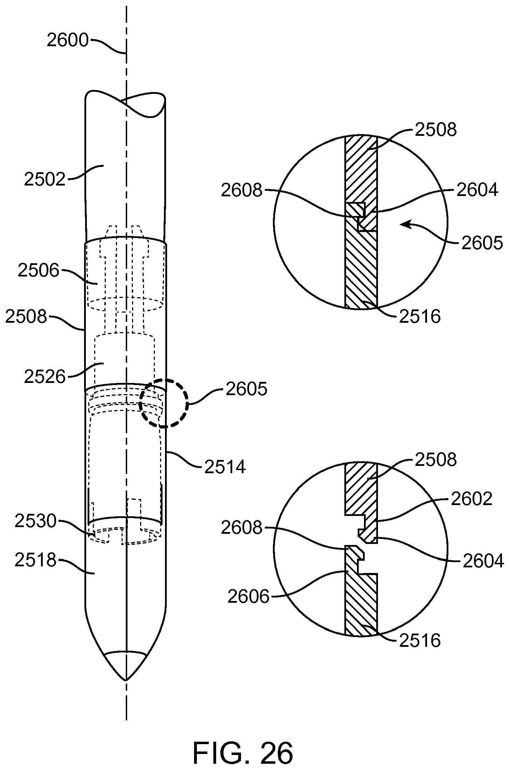

In Figure 25, the tip assembly 2508, 2514 can include a jaw assembly component 2514. The jaw assembly component 2514 can be a member having a proximal end 2516 and a distal end 2518. The proximal end 2516 can be configured to be coupled to the adapter sleeve 2508. The distal end 2518 can be the end that will be inserted to the body cavity. As illustrated in Figure 25, the distal end 2518 of the jaw assembly component 2514 can include at least one jaw 2520, 2522. In Figure 25, the jaw assembly component 2514 can include a pair of jaws 2520, 2522; however, those of ordinary skill in the art will appreciate that fewer or more jaws can be included than as illustrated, so long as the jaw assembly component 2514 includes at least one jaw. In Figure 25, the pair of jaws 2520, 2522 are hingedly coupled to the distal end 2518 of the jaw assembly component 2514. The pair of jaws 2520, 2522 can be hingedly coupled such that the jaw assembly component 2514 has a first position and a second position. While not illustrated in Figures 25-35, those of ordinary skill will appreciate that the first position for jaw assembly component 2514 can be similar to the first position (such as a rest position) and the second position (such as the expanded position), described above with respect to the jawed trocar assemblies illustrated in Figures 1-24. For example, the tip assembly 2508, 2514 can have a first position such that the jaws 2520, 2522 are substantially parallel to a longitudinal axis 2600 (illustrated in Figure 26) of the adapter sleeve 2508 and a second position where at least one of the jaws 2520, 2522 is rotated such that an end of at least one of the jaws 2520, 2522 is positioned radially away from the longitudinal axis 2600. Also as illustrate in Figure 25, one of the jaws 2520, 2522 can have a penetrating member 2524 or penetrating surface a distal end of one of the jaw 2520, 2522. Those of ordinary skill in the art will appreciate that the jaws 2520, 2522 of the jaw assembly component 2508 can be configured in accordance with any of the jaws of the jawed trocars described in relation to and illustrated in Figures 1-24.

Also illustrated in Figure 25, the tip assembly 2508, 2514 can include a locking member 2526 configured to maintain the jaw assembly component 2520 in the first position (such as the rest position). The locking member 2526 can be similar to the locking members described above in relation to Figures 1-23. As illustrated in Figure 25, the locking member 2526 can be insertable into the jaw assembly component 2514 of the tip assembly 2508, 2514. The adapter sleeve 2508 can be configured to fit around the locking member 2526 such that the locking member 2526 is positioned within the adapter sleeve 2508, as will be described further in relation to Figures 26-35.

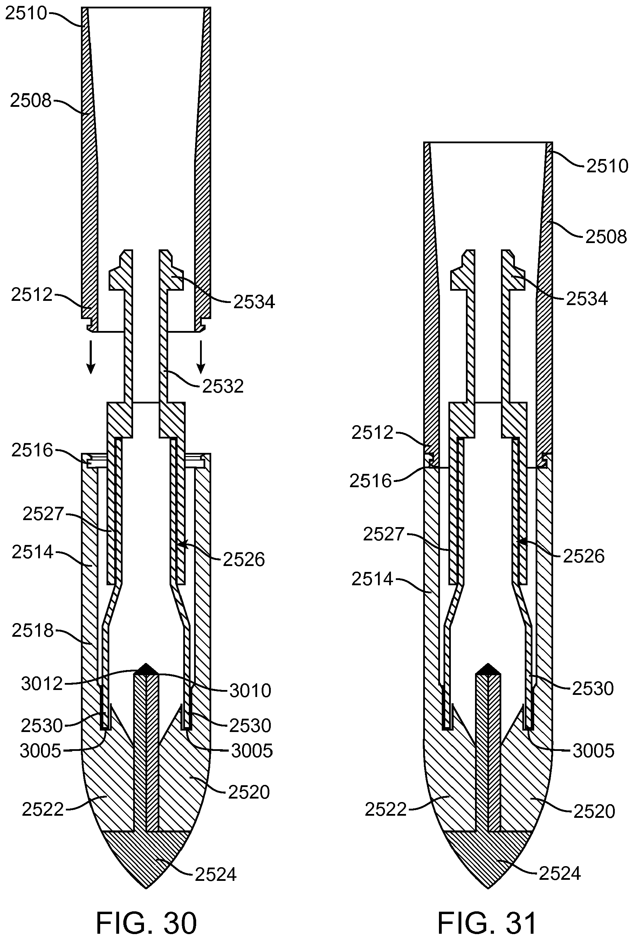

In the particular embodiment illustrated in Figure 25, the locking member 2526 can be a locking collar. The locking member 2526 can be a cylindrical member 2527 having a proximal end 2532 and a distal end 2528. The proximal end 2532 can be received within the adapter sleeve 2508 and the elongate member 2502 of the trocar 2501, when the tip assembly 2508, 2514 is assembled with the trocar 2501. The distal end 2528 can include at least one tab 2530 configured to be received by the jawed component assembly 2514 (for example, by a slot 3005 (shown in Figure 30) formed in an interior portion of the jawed component assembly 2514) such that the at least one tab 2530 maintains the jawed component assembly 2514 in a first or rest position. The tab 2530 can be integrally formed in the distal end 2528 of the locking member 2526 or can be affixed, attached, or otherwise coupled to the distal end 2528 of the locking member 2526. The tab 2530 can be a foot, a peg, or any other protrusion which can be configured to maintain the jawed component assembly 2514 in the first or rest position. In Figure 25, the locking member 2526 includes four tabs 2530 integrally formed on and protruding outwardly from the distal end 2528. Those of ordinary skill in the art will appreciate that fewer or more tabs 2530 can be included in the locking member 2530, so long as the locking member 2526 includes at least one tab 2530.

The proximal end 2532 of the locking member 2526 can include at least one removing member 2534 configured to cooperate with a locking-member-removal tool 2536, such as the one illustrated in Figure 25. The at least one removing member 2534 can be configured to matingly engage with the locking-member-removal tool 2536 such that the locking member 2526 can be disengage from the jawed component assembly 2514, thereby permitting the jawed component assembly 2514 to be transitionable into a second position, such as an expanded position. The at least one removing member 2534 can be integrally formed with, affixed to, attached to, or otherwise coupled to the proximal end 2543 of the locking member 2526. In Figure 25, the locking member comprises two removing member 2534. The two removing members 2534 can be arms which protrude from the proximal end 2527 of the locking member 2526 in a direction towards the proximal end 2504 of the trocar 2501 when the tip assembly 2508, 2514 is assembled with the trocar 2501. The removing members 2534 can be shaped to matingly engage with a corresponding receiving portion of the locking-member-removal tool 2536 such that when the removing member 2534 matingly engages the locking-member removal tool 2536, the locking-member-removal tool 2536 grasps or grips onto the removing member 2534 to remove the locking member 2526 from the jaw assembly component 2514.

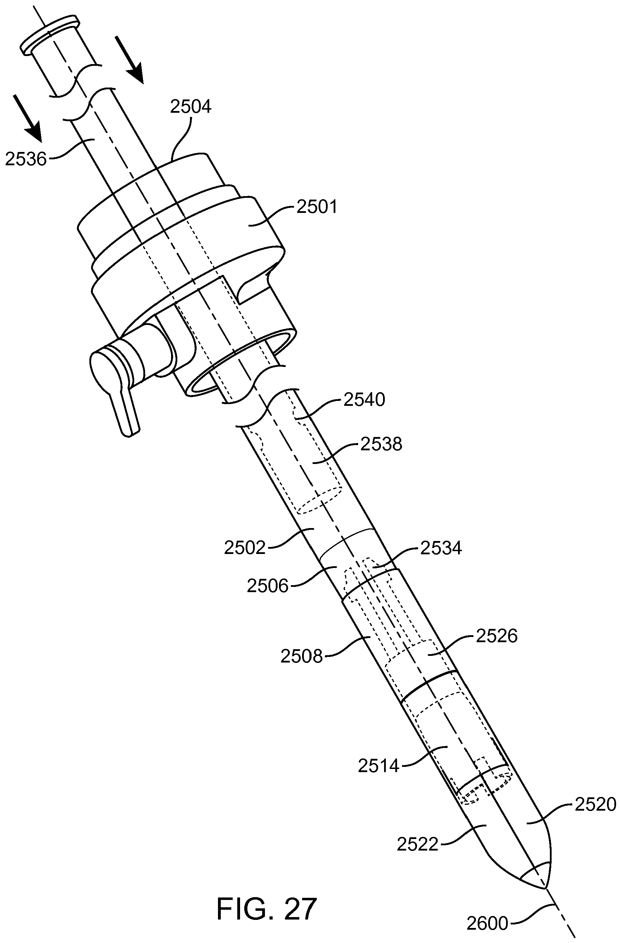

The locking-member-removal tool 2536 can be a member 2536 such as a tubular member, a cylindrical member, or any other elongate member that can be inserted into a trocar 2501 and tip assembly 2508, 2514 to remove a locking member 2526 received by the jaw assembly component 2514 of the tip assembly such that the jaw assembly component 2514 can be transitioned into a second or expanded position. The locking-member-removal tool 2536 can have a distal end 2538 configured to engage the proximal end 2532 of the locking member 2526. The distal end 2538 can be configured to matingly engage the proximal end 2532 of the locking member 2526 such that the locking-member removal tool 2536 grasps or grips the locking member 2526 to disengage the locking member 2526 from the jaw assembly component 2514 in the event the jaw assembly component 2514 receives the locking member 2526. For example, as illustrated in Figure 25, the distal end 2538 of the locking-member removal tool 2536 can define at least one recess 2540 configured to matingly engage the at least one removing members 2534 of the locking member 2526. For example, the at least one recess 2540 can act as a catch to catch, grip, or otherwise securely receive a portion of the removing members 2534, such that when the locking-member-removal tool 2536 is withdrawn or removed from the trocar 2504, the locking member 2526 remains securely coupled to the locking-member-removal tool 2536, which thereby disengages the locking member 2526 from the jaw assembly component 2514 to permit the jaw assembly component 2514 to be transitionable into the second or expanded position. The removal of the locking member 2526 will be discussed in further detail with respect to Figures 27-35.

First, however, this disclosure turns to Figure 26 which illustrates the assembly of the trocar 2501, the tip assembly 2508, 2514, and the locking member 2526. In Figure 26, the proximal end 2516 of the jaw assembly component 2514 can be coupled to a distal end 2512 of the adapter sleeve 2508. In Figure 26, the adapter sleeve 2508 and the jaw assembly component 2514 are configured for mating engagement. For example, the mating engagement 2605 between the adapter sleeve 2508 and the jaw assembly component 2514 is identified by a dashed circle. Close-up views are provided in Figures 26 illustrating the adapter sleeve 2508 and the jaw assembly component 2514 in a mating engagement and a non-mating engagement. As illustrated in Figure 26, the adapter sleeve 2508 includes a mating member 2602 coupled to or formed on the distal end 2512 of the adapter sleeve 2508 and configured to matingly engage a corresponding mating member 2606 coupled to or formed on the proximal end 2516 of the jaw assembly component 2514. For example, the mating member 2602 can include a lip 2604 configured to matingly engage a corresponding rim 2608 of the jaw assembly component 2514. In one embodiment, the mating member 2602 of the adapter sleeve 2508 and the corresponding mating member 2606 of the jaw assembly component 2514 can be a threaded assembly, a snap-engagement assembly, a press-fit assembly, a pin assembly, or any other mating assembly which permits the mating engagement of the adapter sleeve 2508 to the jaw assembly component 2514.

In Figure 26, the proximal end 2510 of the adapter sleeve 2508 can receive a distal end 2506 of the elongate member 2502 of the trocar 2501. For example, the proximal end 2510 of the adapter sleeve 2508 can have a diameter that is larger than the diameter of the distal end 2506 of the trocar 2501 such that the distal end 2506 can be inserted in the proximal end 2510 of the adapter sleeve 2508. In another example, the distal end 2506 can be tapered, such that the distal end 2506 can be inserted in the proximal end 2510 of the adapter sleeve 2508. In Figure 26, the distal end 2506 of the trocar 2501 can be snug-fit, press-fit, adhered, affixed, static-fit, friction-fit, coupled by a ring, or otherwise coupled to the proximal end 2510 of the adapter sleeve 2508 such that the trocar 2501 and adapter sleeve 2508 remain coupled during endoscopic procedures. The adapter sleeve 2508 can be configured such that the adapter sleeve 2508 is removably couplable to the trocar 2501, thereby permitting the interchangeability of the adapter sleeve 2508 with other trocars 2501 and the disposability of the adapter sleeve 2508 after one or more medical or endoscopic procedures. In other embodiments, the adapter sleeve 2508 can remain coupled to the trocar 2501, and the jaw assembly component 2514 can be removable from the adapter sleeve 2508, thereby permitting the interchangeability of the jaw assembly component 2514 with a plurality of trocars 2501. Additionally, the removability of the jaw assembly component 2514 from the adapter sleeve 2508 can permit the disposal of the jaw assembly component 2514 after one or more medical or endoscopic procedures.

Also illustrated in Figure 26, the locking member 2526 can be received by the jaw assembly component 2514. As illustrated in Figure 26, as the locking member 2526 is received by the jaw assembly component 2514, the jaw assembly component 2514 is maintained in the first or rest position, where the jaws of the jaw assembly component 2514 are parallel to the longitudinal axis 2600 of the adapter sleeve 2508 (which can also be the longitudinal axis of the elongate member 2502 of the trocar 2501). As illustrated in Figure 26, when the jaw assembly component 2514 is assembled with the adapter sleeve 2508, the locking member 2526 is located within the adapter sleeve 2508 and the jaw component assembly 2514. Also, in Figure 6, when the trocar 2501, adapter sleeve 2508, and jaw component assembly 2514 are assembled, the locking member 2526 is located interiorly with respect to each of the trocar 2501, adapter sleeve 2508, and jaw component assembly 2514 such that the proximal end 2532 of the locking member 2526 is positioned within an interior of the elongate member 2502 of the trocar 2501.

The removal of the locking member 2526 will now be discussed with respect to Figures 27-29 which are perspective views of a trocar 2501 assembled with the tip assembly 2508, 2514 having a locking member 2526.

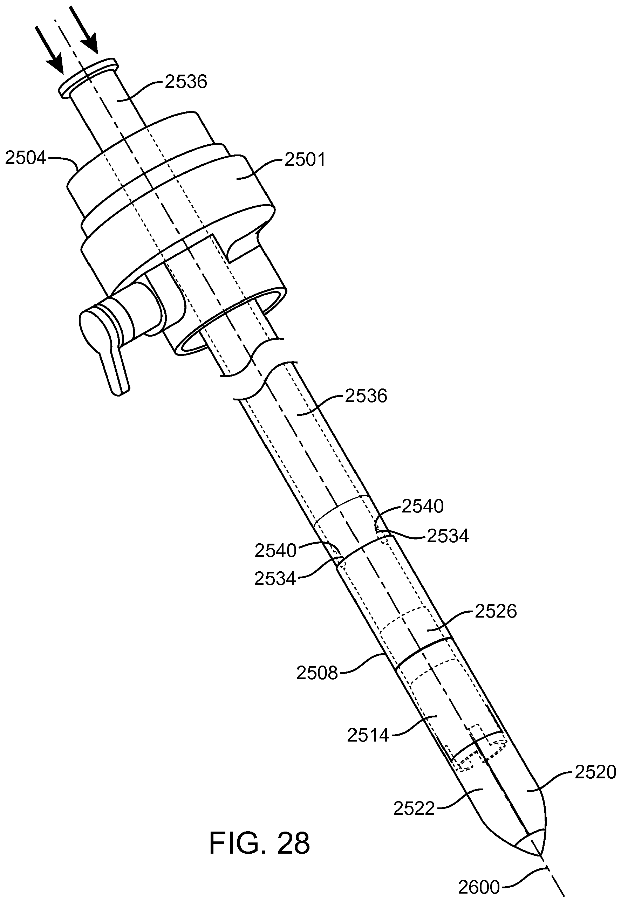

In Figure 27, a locking-member-removal tool 2536 can be inserted through the proximal end 2504 of the trocar 2501. The locking-member-removal tool 2536 can be advanced through the elongate member 2502 of the trocar 2501 until the distal end 2538 of the locking-member-removal tool 2536 engages the proximal end 2532 of the locking member 2526. For example, as illustrated in Figure 28, the locking-member-removal tool 2536 can be advanced such that the distal end 2538 of the locking-member-removal tool 2536 engages the removing members 2534 of the proximal end 2532 of the locking member 2526.

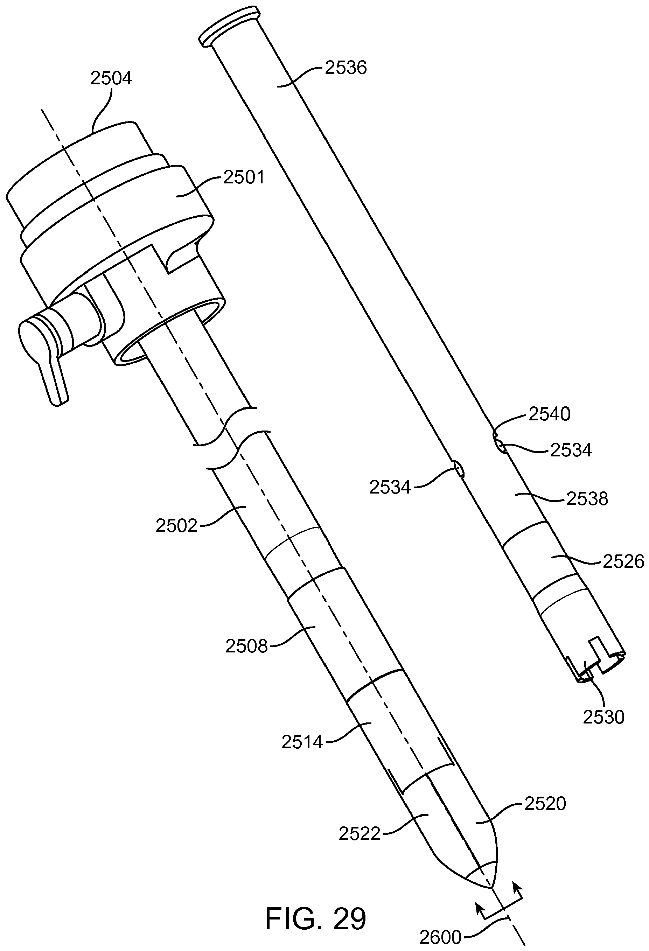

In Figure 28, the recesses 2540 of the locking-member-removal tool 2536 receive the removing members 2534 of the locking member 2526. For example, the removing members 2534 can be biased away from a center of the locking member 2526 such that when the distal end 2538 of the locking-member-removal tool 2536 is advanced towards the removing members 2534, the removing members 2534 are deformed or squeezed toward one another and toward a center of the locking member 2526. Then, as the distal end 2538 of the locking-member-removal tool 2536 can be further advanced toward the locking member 2526, the biasing of the removing members 2534 away from the center of the locking member 2526 can bias the ends of the removing members 2534 such that the ends removing members 2534 catch or are received by a corresponding recess 2540 of the locking-member-removal tool 2536. As the locking-member-removal tool 2536 and the removing members 2534 of the locking member 2526 are now matingly engaged, the locking-member-removal tool 2536 can be withdrawn from the trocar 2501, as illustrated in Figure 29.

In Figure 29, the locking-member-removal tool 2536 can be withdrawn from the trocar 2501 such that the locking-member-removal tool 2536 is removed from the trocar 2501. As illustrated in Figure 29, as the locking-member-removal tool 2536 and the removing members 2534 of the locking member 2526 are matingly engaged, the locking member 2526 is also removed from the trocar 2501, and as a result disengaged and removed from the jaw assembly component 2514 of the tip assembly 2505, 2514, thereby permitting the jaw assembly component 2514 to be transitionable into the second or expanded position for medical or endoscopic procedures.

Figures 30 and 31 are cross-sectional views of the assembly of jaw assembly component 2514 and the adapter sleeve 2508 of the tip assembly 2508, 2514 with the locking member 2526. The assembly of the tip assembly 2508, 2514 and the locking member 2526 is similar to that described with respect to Figure 26, except that the slots 3005 of the jaw assembly component 2514 are more clearly illustrated. As discussed above, the slots 3005 are configured to receive the tabs 2530 of the locking member 2526 such that jaw assembly component 2526 can remain in the first of rest position. Also illustrated in Figure 30, the jaw assembly component 2514 can includes a lens cleaner 3010, 3012 coupled to an interior surface of the jaw assembly component 2514. For example, in Figure 30, the lens cleaner includes a first wiper 3010 and a second wiper 2012, each coupled to one of the first jaw 2520 and the second jaw 2522 of the jaw component assembly 2514. The lens cleaner 2010, 2012 can be a lens cleaner or scope cleaner as discussed above with respect to Figures 1-24.

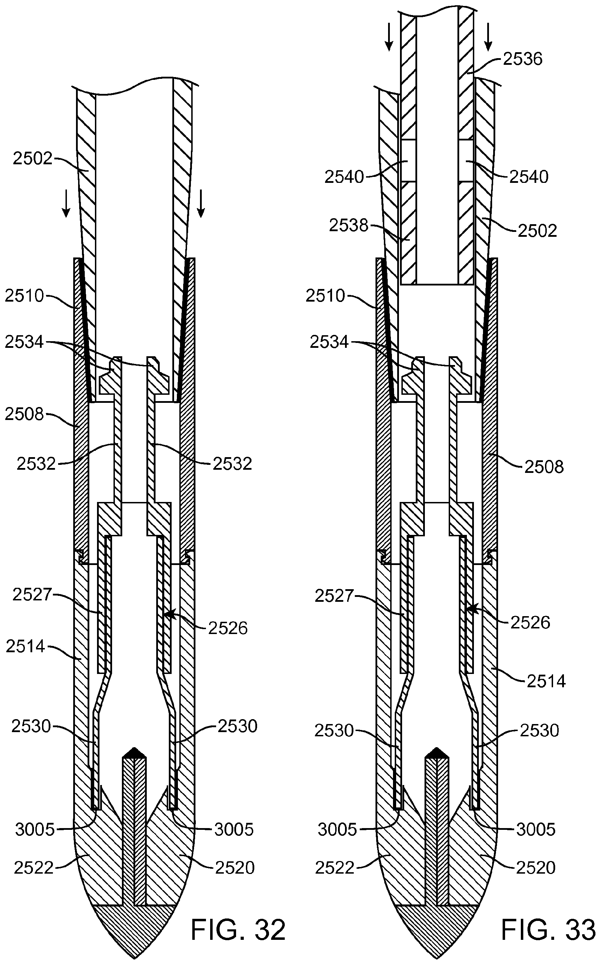

Figure 32 illustrates a cross-sectional view of the assembly of the trocar 2501 with the tip assembly 2508, 2514 assembled in Figures 30 and 31. Specifically, Figure 32 illustrates the insertion of a distal end 2506 of the elongate member 2502 of the trocar 2501 into the proximal end 2510 of the adapter sleeve 2508 of the tip assembly 2508, 2514.

Figures 33-35 illustrates a cross-sectional view of the removal of the locking member 2526 using the locking-member-removal tool 2536, as illustrated in Figures 27-29, except that that the slots 3005 of the jaw assembly component 2514 are more clearly illustrated. While Figures 27-35 describe a tip assembly 2508, 2514 in which the adapter sleeve 2508 and the jaw assembly component 2514 are matingly engaged, those of ordinary skill in the art will appreciate that the adapter sleeve 2508 and the jaw assembly component 2514 can be integrally formed. Those of ordinary skill in the art will appreciate that the adapter sleeve 2508 and jaw assembly component 2514 can be substantially transparent to allow light to pass therethrough so that an image can be captured by a camera within the trocar assembly.

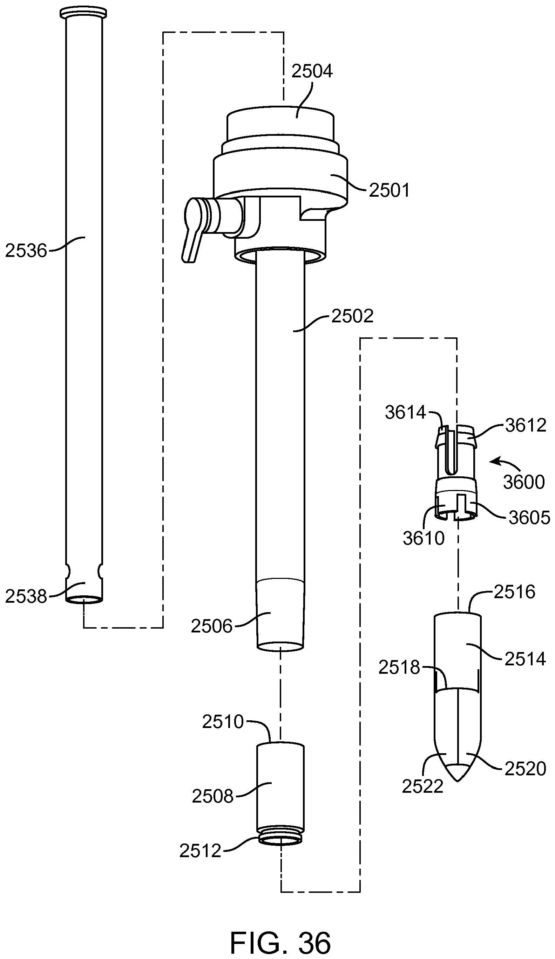

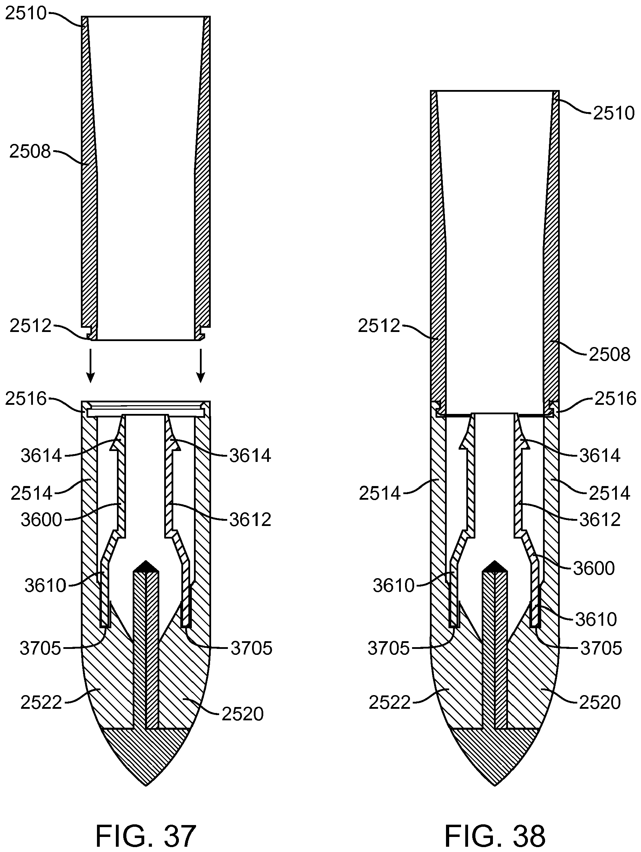

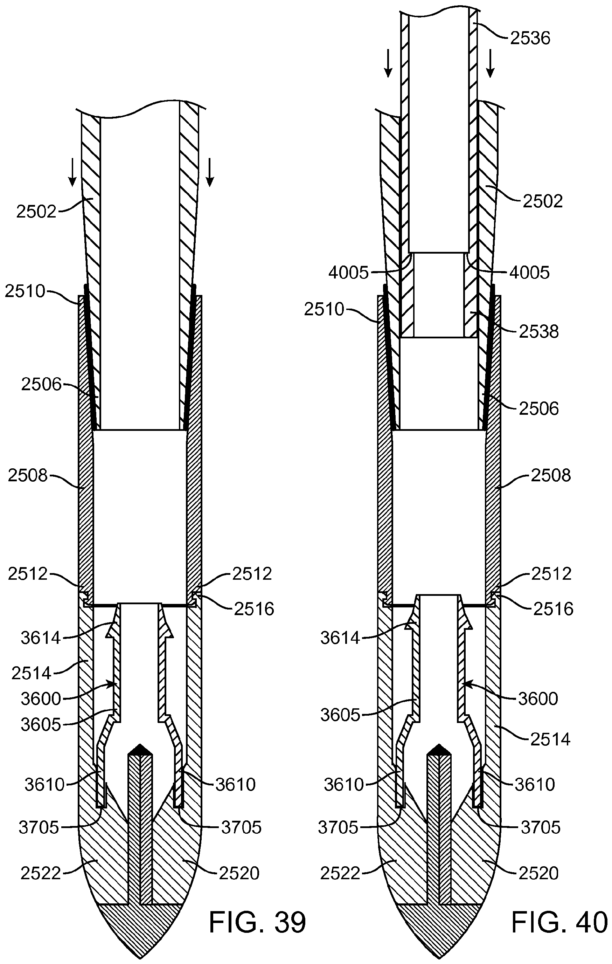

Figure 36 is an exploded view of a trocar 2501 and tip assembly 2508, 2514 similar to that illustrated in Figure 25, except that the locking member 3600 and locking-member-removal tool 2536 are different than as illustrated in Figure 25. In Figure 36, the locking member 3600 is shorter in length than the locking member 2526 illustrated in figure 25. Similar to the locking member 2526 illustrated in Figures 25, the locking member 3600 illustrated in Figure 36 includes a hollow member having a distal end 3605 and a proximal end 3612. The distal end 3605 of the locking member 3600 can includes at least one tab 3610 configured to engage corresponding slots 3705 (shown in Figure 37) of the jaw assembly component 2514. As discussed above, the slots 3705 can be formed in an interior surface of the jaw assembly component 2514. In Figure 36, the locking member 3600 includes four tabs 3610 integrally formed and protruding away from a center of the locking member 3600 towards the jaw assembly component 2514. In Figure 36, the proximal end 3612 of the locking member can include four removing members 3614 integrally formed on the proximal end 3612 of the locking member 3600 and protruding away from the center of locking member 3600 towards the proximal end 2504 of the trocar 2501. Similar to the locking member 2526 illustrated in Figure 25, the removing members 3614 can be configured to matingly engage an interior surface 4005 (shown in Figure 40) of the locking-member-removal tool 2536.

The disclosure now turns to Figures 37-38 that illustrate a cross-sectional view of the assembly of the tip assembly 2508, 2514, For example, Figures 37-38 illustrate that the proximal end 2516 of the jaw assembly component 2514 can be configured to receive the distal end 2512 of the adapter sleeve 2508 via a threaded assembly, a snap-fit assembly, a press-fit assembly, or any other coupling configured to couple the jaw assembly component 2514 with the adapter sleeve 2508. The assembly of the jaw assembly component 2514 with the adapter sleeve 2508 is substantially similar to that discussed above in relation to Figures 30 and 31, except that the tabs 3610 locking member 3600 are configured to be received by recess 3705 formed in an interior space of the jaw assembly component 2502 such that the locking member 3600 is positioned within an interior space of the jaw assembly component, rather than an interior space of the adapter sleeve 2508 and/or an elongate member 2502 of a trocar 2501 assembled with the tip assembly 2508, 2514. As illustrated in Figures 37-38, the ends of the removing members 3614 of the proximal end 3512 of the locking member 3600 is positioned with the interior space of the jaw assembly component 2514.

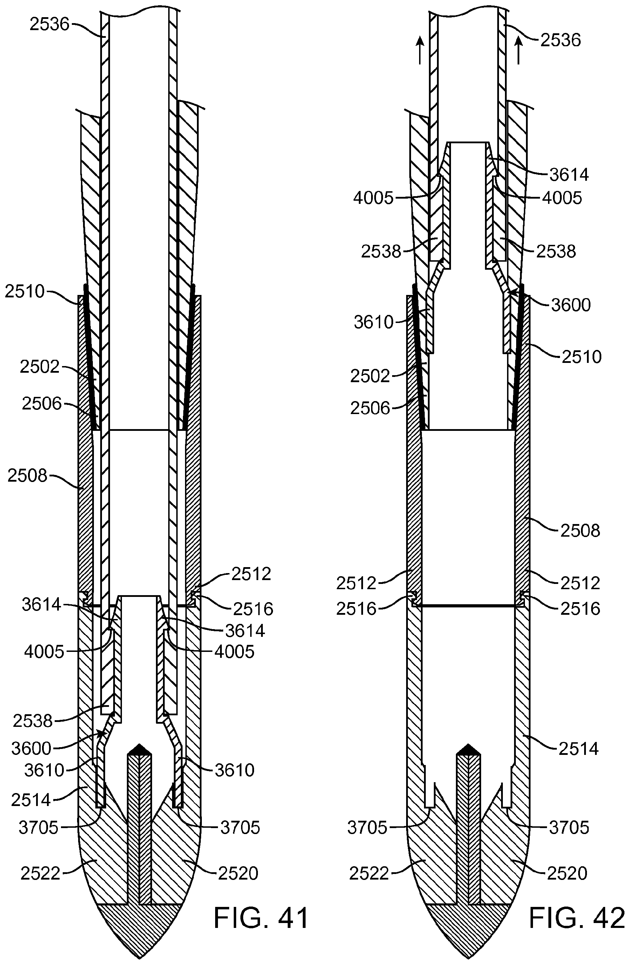

Figures 39-42 illustrate a cross-sectional view of the removal of locking member 3600 from the slots 3705 of the jaw assembly component 2514, thereby permitting the transition of the jaw assembly component 2514 from the first or rest position to the second position or expanded position. In Figure 39, a distal end of the elongate member 2502 of the trocar 2501 can be inserted in the adapter sleeve 2508 of the tip assembly 2508, 2514. In Figure 40, the locking-member-removal tool 2536 can be inserted through the trocar 2501 an advanced through the elongate member 2502, the adapter sleeve 2508, and the jaw assembly component 2514, until the distal end 2530 of the locking-member-removal tool 2536 engages a proximal end 3612 of the locking member 3600. Similar to Figures 33-35, in Figures 40-42, the removing members 3614 of the locking member 3600 can be biased away from a center of the locking member 3600 such that when the distal end 2538 of the locking-member-removal tool 2536 is advanced towards the removing members 2534, the removing members 3614 are deformed or squeezed toward one another and toward a center of the locking member 2600. Then, as the distal end 2538 of the locking-member-removal tool 2536 can be further advanced toward the locking member 3600, the biasing of the removing members 3614 away from the center of the locking member 3600 can bias the ends of the removing members 2534 such that the ends removing members 2534 catch or are received by a corresponding portion 4005 of the locking-member-removal tool 2536, as illustrated in Figure 41. For example, as illustrated in Figures 40-42, the corresponding portion 4005 can be an interior edge, a ledge, a protruding rim, or other portion configured to engage the removing members 3614 of the locking member 3600, such that the withdrawn of the locking-member-removal tool 2536 includes the removal of the locking member 3600 from engagement with the jaw assembly component 2514, for example, as illustrated in Figure 42. In Figure 41, the removing member 3614 of the locking member 3600 engage the corresponding portion 4005 (an inner ledge formed along an interior surface of the jaw assembly component 2514) and are biased outwardly away from the center of the locking member 3600 such that the removing members 3614 a substantially prevented from disengaging from the locking-member-removal tool 2536. With the locking member 3600 matingly engaged with the locking-member-removal tool 2536, the locking member 3600 matingly engaged with the locking-member-removal tool 2536 can be removed from the trocar 2501, thereby permitting the jaw assembly component 2514 to be transitioned from the first or rest position into a second or expanded position, for example, as illustrated in Figure 42.

In Figure 42, the locking-member-removal tool 2536 is withdrawn from the tip assembly 2505, 2514 and from the elongate member 2502 of the trocar 2501. The locking member 3600 can be coupled the distal end 2530 of the locking-member removal tool 2536 as a result of the mating engagement between the removing members 3614 and the corresponding portion 4005 of the locking-member-removal tool 2536. Thus, as the locking-member removal tool 2536 is withdrawn, retracted, or otherwise removed from the trocar 2501, the locking member 3600 is also withdrawn, retracted, or otherwise removed from the trocar 2501, thereby permitting the jaw assembly component 2514 to be transitionable between the first or rest position and the second or expanded position for medical or endoscopic procedures.

Similar to Figures 25-35, the tip assembly 2505, 2514 illustrated in Figures 36-42 can be removably coupled to the trocar 2501 of a trocar assembly, thereby permitting the interchangeability of the tip assembly 2505, 2514 with a plurality of trocars 2501 and the disposability of the tip assembly 2505, 2514 after one or more medical or endoscopic procedures. Additionally, the adapter sleeve 2508 can remain coupled to the trocar 2501, and the jaw assembly component 2514 can be removable from the adapter sleeve 2508, thereby permitting the interchangeability of the jaw assembly component 2514 with a plurality of trocars 2501. Additionally, the removability of the jaw assembly component 2514 from the adapter sleeve 2508 can permit the disposal of the jaw assembly component 2514 after one or more medical or endoscopic procedures.

While Figures 25-42 illustrate specific embodiments of a removable tip assembly 2505, 2514 removably coupled to distal ends 2506 of trocars 2501, those of ordinary skill in the art will appreciate that the various components and features disclosed herein with respect to Figures 1-42 can be interchanged and optionally included to achieve the technical advantages and benefits of the jawed trocar assembly disclosed herein. For example, the removable tip assembly 2505, 2514 can include a reflecting member as described above in relation to Figures 22 and 23. In one example, the reflecting member can be coupled to or integrally formed with the locking member 2526 removably coupled to the jaw assembly component 2526 of the tip assembly 2505, 2514.

In another embodiment, another jaw retention device can be implemented either in place of the locking member or in addition to the locking member. When implemented in addition to the locking member, the jaw retention device further provides for an additional mechanism to hold the jaws in a closed configuration. The closed configuration is used during the insertion of the tip assembly and the associated trocar.

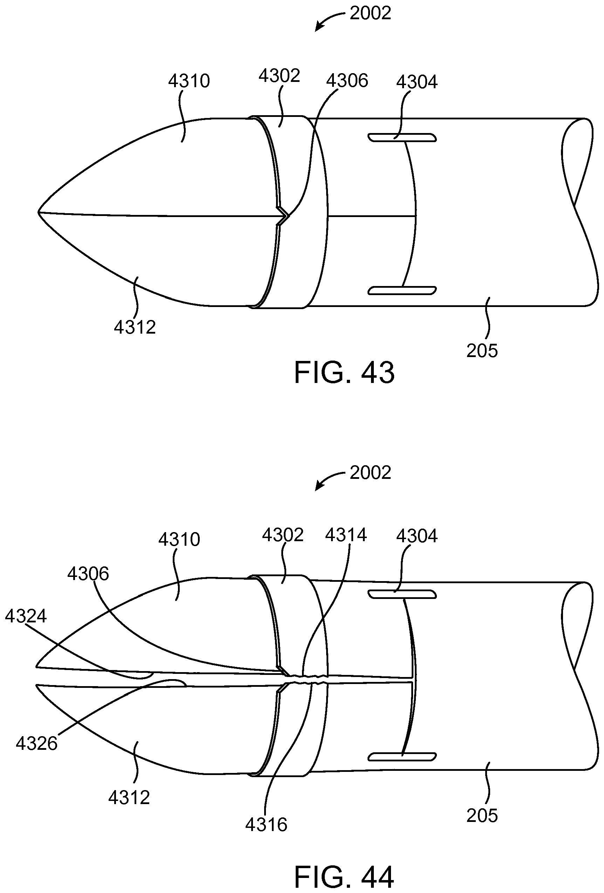

One example of another jaw retention device is illustrated in Figure 43, which is a side elevation view of an exemplary tip assembly 2002, in a closed configuration, having an exemplarily jaw retention device in the form of a band 4302. The band 4302 can be configured to encircle the jaws 4310, 4312. When the band 4302 encircles the jaws 4310, 4312, the band 4302 holds the jaws 4310, 4312 in a closed configuration such that lower jaw 4312 substantially abuts the upper jaw 4310. As used herein, substantial abutment of the lower jaw 4312 with the upper jaw 4310 indicates a close fit such that there can be a small space between the lower jaw 4312 and the upper jaw 4310 or almost no space such that the lower jaw 4312 is pressed against the upper jaw 4310. The substantial abutment of the lower jaw 4312 and upper jaw 4310 is a close fit so as to allow tip assembly 2002 to penetrate as described herein without tearing tissue. The band 4302 includes two notched portions 4306 (the other notched portion is on the reverse side). The notched portion 4306 is configured to promote fracturing of the band 4302 at substantially the region of the band 4302 that overlaps the region where the upper jaw 4310 and the lower jaw 4312 abut one another.

The band 4302 can be configured in a variety of ways to facilitate the implementation with the tip assembly. For example, the edges of the band 4302 can be tapered so as to provide a smooth transition from the jaws 4310, 4312 to the band 4302. In at least one implementation, the band 4302 can be co-molded with the first jaw 4310 and the second jaw 4312, for example using a two shot molding process. In another implementation, the band 4302 can be include an adhesive such that the band 4302 is slipped over the tip of the tip assembly 2002 and pressed into position. In yet another embodiment, the band 4302 can be an adhesive tape that is wrapped around the first jaw 4310 and the second jaw 4312. When the band 4302 is a tape, the seam of the tape can be either on the first jaw 4310 or the second jaw 4312 away from the portion of the jaws 4310, 4312 that abut one another. The tape can include notched portions 4302 as illustrated.

The band 4302 can be fractured thereby allowing the upper jaw 4310 to separate from the lower jaw 4312. In order to fracture the band 4302, an implement 400 such as an endoscope or camera implement as described herein. The jaws 4310, 4312 can be configured to operate as described herein above. For example, the jaws 4310, 4312 can be configured to open once the band 4302 has been fractured. In another example, the jaws 4310, 4312 are only opened in relation to how far the implement 400 is extended through the jaws 4310, 4312 such that the jaws 4310, 4312 are only fully opened when the implement 400 has been extended so far as to cause the jaws to reach their fully open configuration. In yet another embodiment, a specially designed jaw opening implement can used to facture the band 4302.

Figure 44 is a side elevation view of the exemplary tip assembly 2002 of Figure 43, in an open configuration. As illustrated in Figure 44 the band 4302 has been fractured, when the band 4302 has been fractured the jaws 4310, 4312 can be configured to open as indicated above. As seen if Figure 44, the band 4302 has a first fracture surface 4314 and a second fracture surface 4316. The first fracture surface 4314 can substantially align with an edge 4324 of the upper jaw 4310. The second fracture surface 4316 can substantially align with an edge 4326 of the lower jaw 4312. As indicated above, the band 4302 fractures in two places with one of those places being on the reverse side of the illustration. While the illustrated jaws 4310, 4312 essentially form a half of the tip assembly 2002 over the relevant region, the jaws 4310, 4312 could be configured in other ways such that they are not equally divided.

In order to assist with the opening of the jaws 4310, 4312, the tip assembly 2002 can be provided with slots 4304 which allow the jaws 4310, 4312 to move in relation to one another without distorting or binding the material of the tip assembly 2002. In another embodiment the slots 4304 can reduce the distortion or binding of the material of the tip assembly 2002. In at least one embodiment, four slots 4304 are provided on the tip assembly 2002. In another embodiment, only two slots 4304 can be provided. In yet another embodiment, any multiple of two slots can be implemented.

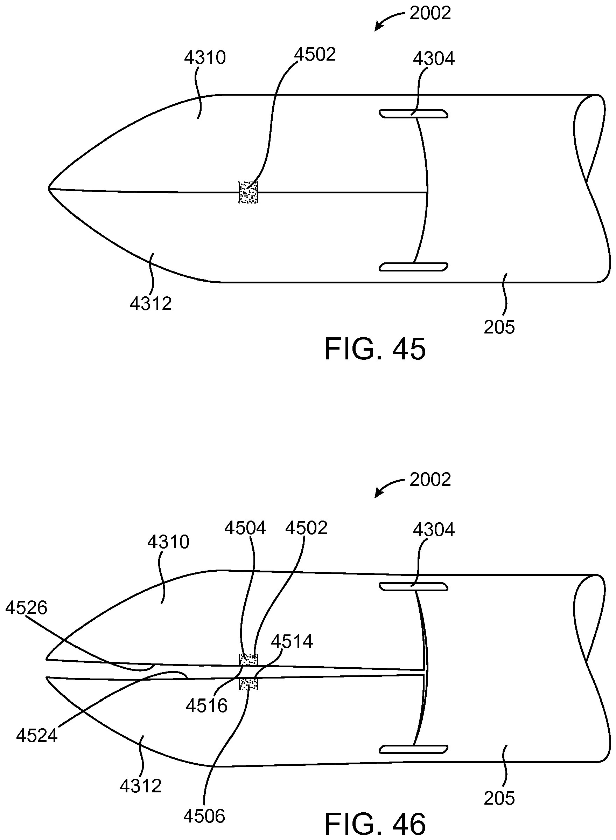

Another embodiment of an exemplary tip assembly 2002 is illustrated in Figure 45. The illustration of tip assembly 2002 in Figure 45 is a side elevation view of a closed tip assembly 2002 having an exemplarily jaw retention device in the form of at least one tab 4502. The at least one tab 4502 can be located at the region where the upper jaw 4310 and lower jaw 4312 substantially abut one another. In at least one embodiment, the at least one tab 4502 can be at least two tabs 4502. In other embodiments, a plurality of tabs 4502 can be implemented. For example, multiple tabs can be implemented on each side of the illustrated embodiment.

The description herein refers to tabs 4502 as in the illustrated embodiment, two tabs 4502 are implemented (one not shown). The description as provided herein can be applied to other configurations of tabs 4502 as well. The tabs 4502 can be configured to be co-molded with the upper jaw 4310 and the lower jaw 4312 such that during the molding process the tabs 4502 connect the upper jaw 4310 with the lower jaw 4312. The tabs 4502 can be integrally formed with the upper jaw 4310 and the lower jaw 4312 such that no portion of the tabs 4502 extend beyond the outer surfaces of the upper jaw 4310 and the lower jaw 4312. For example, the tabs 4502 can be formed such that they are within the width of material forming the upper jaw 4310 and the lower jaw 4312. In another embodiment the tabs 4502 can be formed on the inside of the upper jaw 4310 and the lower jaw 4312. However, in at least one embodiment the tabs 4502 can be formed on the outside of the upper jaw 4310 and the lower jaw 4312. When the tabs 4502 are formed on the outside of the upper jaw 4310 and the lower jaw 4312, the tabs 4502 can be tapered so as to provide a smooth transition from the upper jaw 4310 and the lower jaw 4312 to the thickest portion of the tabs 4502.

While not illustrated, the tabs 4502 can include notched portions to facilitate the fracturing of the tabs 4502. In other embodiments, the tabs may not include notched portions, but instead the material of the tabs 4502 can be choose such that it facilities easy fracturing but is strong enough to hold the jaws 4310, 4312 in place during insertion of the tip assembly 2002. ;

Figure 46 is a side elevation view of the exemplary tip assembly of Figure 45, in an open configuration, wherein the at least one tab 4502 has been fractured. The at least one tab 4502 can be fractured thereby allowing the upper jaw 4310 to separate from the lower jaw 4312. In order to fracture the at least one tab 4502, an implement 400 such as an endoscope or camera implement as described herein. The jaws 4310, 4312 can be configured to operate as described herein above. For example, the jaws 4310, 4312 can be configured to open once at least one tab 4502 has been fractured. In another example, the jaws 4310, 4312 are only opened in relation to how far the implement 400 is extended through the jaws 4310, 4312 such that the jaws 4310, 4312 are only fully opened when the implement 400 has been extended so far as to cause the jaws to reach their fully open configuration. In yet another embodiment, a specially designed jaw opening implement can used to facture the at least one tab 4502.

When the tab 4502 has been fractured as illustrated in Figure 46, the jaws 4310, 4312 can be configured to open as indicated above. As seen if Figure 46, the tab 4502 has a first fracture surface 4516 and a second fracture surface 4514. The first fracture surface 4516 can substantially align with an edge 4526 of the upper jaw 4310. The second fracture surface 4314 can substantially align with an edge 4324 of the lower jaw 4312. While not illustrated, another tab 4502 on the opposite side can be included and can fracture in a similar manner. When another number of tabs 4502 are included the fracturing of the other tabs 4502 can proceed in a similar manner. While the illustrated jaws 4310, 4312 essentially form a half of the tip assembly 2002 over the relevant region, the jaws 4310, 4312 could be configured in other ways such that they are not equally divided.

In order to assist with the opening of the jaws 4310, 4312, the tip assembly 2002 can be provided with slots 4304 which allow the jaws 4310, 4312 to move in relation to one another without distorting or binding the material of the tip assembly 2002. In another embodiment the slots 4304 can reduce the distortion or binding of the material of the tip assembly 2002. In at least one embodiment, four slots 4304 are provided on the tip assembly 2002. In another embodiment, only two slots 4304 can be provided. In yet another embodiment, any multiple of two slots can be implemented.

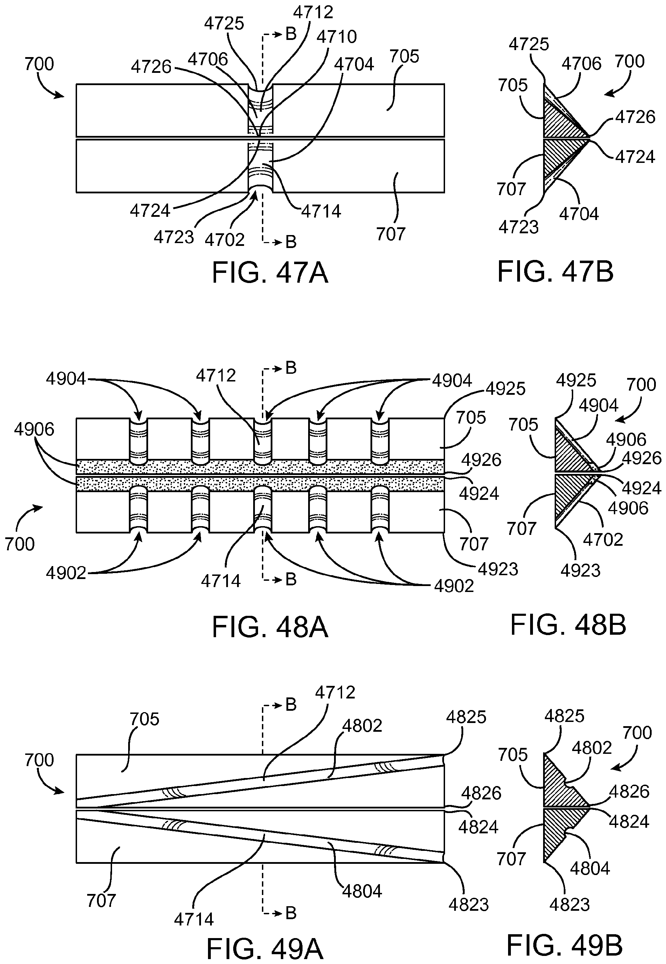

Figures 47A, 47B, 48A, 48B, 49A, and 49B illustrate exemplarily surfaces of scope cleaner 700 according to various embodiments as presented herein. The scope cleaner can include one or more scope cleaners. For example, the scope cleaner 700 can include one or more wiper blades 705, 707. While the illustrated embodiments include two wiper blades 705, 707, at least one embodiment includes only a single wiper blade. Furthermore, while the embodiments illustrated are wiper blades 705, 707, the disclosure as presented herein can equally apply to other types of scope cleaners 700. The one or more grooves illustrated in Figures 47A, 47B, 48A, 48B, 49A, and 49B can be configured to facilitate the flow of fluid as the scope cleaner 700 cleans fluid from the scope that the scope cleaner comes into contact. The surfaces illustrated herein are the surface that faces the scope and at least a portion of the scope cleaner 700 contacts the scope as described above.

A first example is illustrated in Figure 47A which is a top view of an exemplarily scope cleaner 700 having a groove 4702 formed therein. The groove 4702 as illustrated can traverse through a center 4710 of the scope cleaner 700. In the illustrated embodiments, the center 4710 of the scope cleaner 700 refers to midpoint of the scope cleaner 700 in both the lateral and transverse directions (length and width). In other embodiments, the center 4710 of the scope cleaner can be based on centroid of the surface of the scope cleaner 700. In other embodiments, the groove 4702 does not traverse through the center of the scope cleaner 700.

In Figure 47A, the groove 4702 traverses through the center 4710 of the first scope cleaner in the form of a first wiper blade 705 and the center 4710 of the second scope cleaner in the form of a second wiper blade 707. As shown, the groove 4702 comprises a first groove 4706 in the first wiper blade 705 and a second groove 4704 in the second wiper blade 707. Thus, the first wiper blade 705 and the second wiper blade 707 each have at least one groove formed therein. In other embodiments, more than one groove can be present on each of the first wiper blade 705 and the second wiper blade 707.

A cross-sectional view of the scope cleaner 700 along line B-B of Figure 47A is illustrated in Figure 47B. When a first groove 4706 and a second groove 4704 are implemented as illustrated in Figure 47A, the first groove 4706 and the second groove 4704 facilitate the movement of fluid into the groove as the scope is cleaned. When the scope includes two cameras such as the one illustrated in Figures 7 and 9-11, having the groove in the center of the scope cleaner prevents buildup of fluid in the regions in which the camera is mounted. Additionally as illustrated in the example of Figure 47B, the first groove 4706 can be sloped from an inner edge 4726 to an outer edge 4725, such that the depth of the groove 4706 is shallower or non-existent at the inner edge 4726 and deeper at the outer edge 4725. The slope of the first groove 4706 allows for fluid to build up and run down the groove 4706. By having the groove 4706 very shallow or non-existent at the inner edge insures contact with the scope across the entire face of the scope so as to remove all fluid buildup on the scope. Thus, the first groove 4706 allows fluid to be removed from the scope and channeled to an appropriate place to minimize the impact of fluid impinging upon the scope. Furthermore, the first groove 4706 allows the first scope cleaner 707 to be used multiple times to remove fluid from the scope.

Similarly, the second scope cleaner 707 can have a cross-section that is substantially a mirror image of the cross-section of the first scope cleaner 705. The second groove 4704 of the second scope cleaner 707 can be sloped in a similar fashion as described above. For example, the second groove 4704 can be sloped from an inner edge 4724 to an outer edge 4723, such that the depth of the second groove 4704 is shallower or non-existent at the inner edge 4724 and deeper at the outer edge 4723. The slope of the second groove 4704 allows for fluid to build up and run down the second groove 4704. By having the second groove 4704 very shallow or non-existent at the inner edge 4724 insures contact with the scope across the entire face of the scope so as to remove all fluid buildup on the scope. Thus, the second groove 4704 allows fluid to be removed from the scope and channeled to an appropriate place to minimize the impact of fluid impinging upon the scope. Furthermore, the second groove 4704 allows the second scope cleaner 705 to be used multiple times to remove fluid from the scope.

Figure 48A is a top view of another exemplarily scope cleaner having a groove formed therein, according to an exemplarily embodiment. Figure 48B is a cross-sectional view of the scope cleaner of Figure 48A taken along line B-B. As illustrated in Figure 48A, the scope cleaner has a plurality of grooves formed therein. As illustrated there are five grooves 4904 formed in the first scope cleaner, which is in the form of a first wiper 705. Additionally, there are five grooves 4902 formed in the second scope cleaner, which is in the form of a second wiper 705. At least one of the grooves 4904 on the first wiper 705 traverses the center 4712 of the first wiper 705. Similarly, at least one of the grooves 4902 on the second wiper 707 traverses the center 4714 of the second wiper 707.

The scope cleaner 700 as illustrated in Figure 48A further includes an interior portion 4906 that is composed of a different material from the rest of the scope cleaner 700. The interior portion can be configured to provide for an enhanced cleaning of the scope while preventing permeation of fluid. This configuration encourages the flow of the fluid into the grooves 4902, 4904 of the scope cleaner 700.

As seen in the section view of Figure 48B, the inner portion 4906 of the first wiper 705 borders the inner edge 4926 of the first wiper 705. Additionally, the inner portion 4906 of the second wiper 707 borders the inner edge 4924 of the second wiper 707. The grooves 4902, 4904 have a uniform depth. However, in other embodiments the grooves 4902, 4904 can have a sloped depth such that the grooves are like that of Figure 47A and 47B. As the inner portion 4906 is configured to prevent fluid permeation, the fluid drains down the grooves 4902, 4904 away from the newly exposed portion of the scope.

Figure 49A is a top view of yet another exemplarily scope cleaner having a groove formed therein, according to an exemplarily embodiment; and Figure 49B is a cross-sectional view of the scope cleaner of Figure 49A taken along line B-B. As illustrated in Figure 49A, the grooves are arranged along a diagonal direction on the face of the scope cleaner 700. As illustrated the first groove 4802 on the first wiper 705 is arranged along a diagonal line that runs from an inner edge 4826 to an outer edge 4825 of the first wiper 705. The first groove 4802 traverses through the center 4712 of the first wiper 705. As illustrated the second groove 4804 on the second wiper 707 is arranged along a diagonal line that runs from an inner edge 4824 to an outer edge 4823 of the second wiper 707. The second groove 4804 traverses through the center 4714 of the second wiper 707. The grooves operate in a similar fashion to the grooves as described above. Namely, the grooves 4802, 4804 provide for draining of fluid away from the inner edge 4826, 4824 of the respective first and second wiper blades 705, 707. In the cross-sectional view of Figure 49B, the grooves 4802, 4804 have a uniform depth.

While the section profiles have illustrated the wipers as being triangular in shape, the section profile can be other shapes as well. For instance, the section profile can have a parallelogram shape, including rectangular or square shape. When the triangular shape is implemented enhanced cleaning effect of the lens can be achieved.

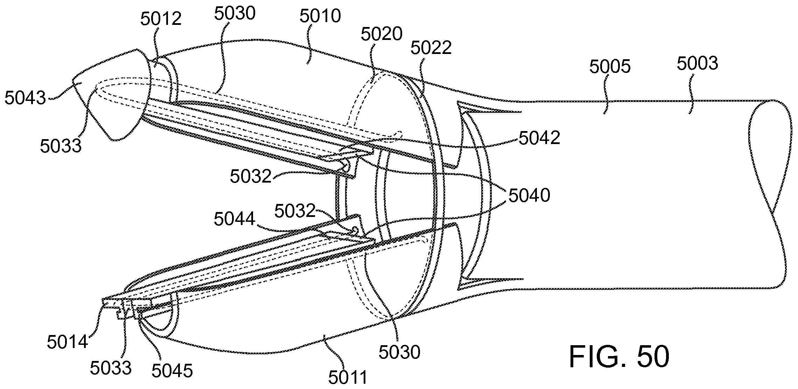

Figures 50-54 illustrate further examples of tip assemblies that can be implemented with the presented technology. The tip assemblies illustrated in Figures 50-54 can be implemented as a part of a trocar or a separate component that is configured to be coupled to a trocar. For example, the tip assemblies can be coupled to the trocar by an adapter sleeve (not shown). The tip assemblies illustrated in Figures 50-54 are illustrated without all of the components in order to aid in illustration. Furthermore, the tip assemblies can be configured to include one or more of the components, elements, functions or features as described herein.

Figure 50 illustrates an exemplarily tip assembly 5005. The exemplarily tip assembly 5005 includes jaws 5010, 5011. An upper jaw 5010 can be configured to flex relative to the body 5003 of the tip assembly 5005. A lower jaw 5011 can also be configured to flex relative to the body 5003. The upper jaw 5010 can include a first slider 5012 that is slidingly coupled to the upper jaw 5010. The slidingly coupling can be through a one or more channels formed on the first slider; the one or more channels can be configured to matingly engage and slide relative corresponding receiving portions formed in the upper jaw 5012. In other embodiments, other configurations of the first slider 5012 and upper jaw 5010 are possible to allow relative motion of the first slider 5012 to the upper jaw 5010. The lower jaw 5011 can include a second slider 5014 that is slidingly coupled to the lower jaw 5011. The slidingly coupling can be through a one or more channels formed on the second slider; the one or more channels can be configured to matingly engage and slide relative corresponding receiving portions formed in the lower jaw 5011. In other embodiments, other configurations of the second slider 5014 and lower jaw 5011 are possible to allow relative motion of the second slider 5014 to the lower jaw 5011.

The upper jaw 5010 and lower jaw 5011 can be configured to flex relative to the body 5003, when a camera or other instrument abuts the upper jaw 5010 and lower jaw 5011. The first slider 5012 can have a wiper 5040 affixed to a first end 5042. The second slider 5014 can have a wiper 5040 affixed to a first end 5044. Thus, as the instrument abuts the wipers 5040, the upper jaw 5010 can separate from the lower jaw 5011. As the upper jaw 5010 separates from the lower jaw 5011, the first slider 5012 moves outward from the upper jaw 5010 and the second slider 5014 moves outward from the lower jaw 5014, thereby forming an opening between the upper jaw 5010 and lower jaw 5011 through which the instrument can pass. The wipers 5040 can be configured as described herein.

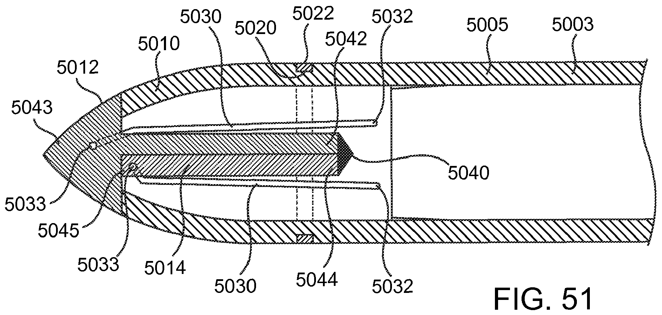

Additionally, a closing mechanism 5022 can be included in the tip assembly 5005. The closing mechanism 5022 can be configured to bias the upper jaw 5010 and lower jaw 5011 to a closed configuration as illustrated in the cross-sectional view of Figure 51. The closing mechanism 5022 in the illustrated embodiment is an elastic band. The elastic band can be metallic, plastic, or rubber. The elastic band can be configured to deliver a desired clamping force to the upper jaw 5010 and lower jaw 5011 so that the upper jaw 5010 and lower jaw 5011 close relative to one another to be in a closed position in which the upper jaw 5010 and lower jaw 5011 substantially abut one another. The closing mechanism 5022 can be configured to rest in a groove 5020 formed in the upper jaw 5010 and lower jaw 5011. The groove 5020 can be configured such that an outer surface of the closing mechanism 5022 is substantially flush with the outer surface of the upper jaw 5010 and lower jaw 5011.

The exemplarily tip assembly 5005 can further include slider biasing members 5030 that bias the first slider 5012 and the second slider 5014 to a retracted configuration relative to the upper jaw 5010 and lower jaw 5011, respectively. As illustrated the biasing member 5030 can be a single component for the first slider 5012 and a single component for the second slider 5014. In other embodiments, the biasing member 5030 can be made of one or more components. For instance, the first slider 5012 can have two biasing members. In yet other embodiments, the number of biasing members 5030 for the first slider 5012 can be one or any other number.

As illustrated the biasing member 5030 can include one or more attachment ends 5032 that are configured to be coupled to the upper jaw 5010 or lower jaw 5011. The attachment ends 5032 can be coupled to the respective jaw 5010, 5011 so that the biasing member 5030 can be releasably affixed to the respective jaw 5010, 5011. In yet other embodiments, the biasing member 5030 can be permanently affixed to the respective jaw 5010, 5011.

As illustrated the biasing member 5030 that passes through the first slider 5012 is configured to pass through a distal end 5043 of the first slider 5012 such that there is an distal portion 5033 of the biasing member 5030 that is nearest to the distal end 5043 of the first slider 5012. Similarly, the biasing member 5030 that passes through the second slider 5014 is configured to pass through a distal end 5045 of the second slider 5014 such that there is a distal portion 5033 of the biasing member 5030 that is nearest to the distal end 5045 of the second slider 5014. In other embodiments, the biasing member 5030 can have different configurations of a distal portion 5033 that can be further away from the distal end 5043, 5045 of the respective slider 5012, 5014. For example, the biasing member 5030 can run less than three-fourths the length of the slider 5012, 5014. In yet other embodiments the biasing member 5030 can run less than half the distance of the slider 5012, 5014. In still other embodiments, the length of the biasing member 5030 can be distance that is selected to provide the appropriate return force and allow for the appropriate opening so that the instrument can pass between the upper jaw 5010 and lower jaw 5011. As indicated above, the biasing member 5030 can be configured to return the sliders 5012, 5014 to a retracted position once the instrument has been removed. Other configurations of the biasing members 5030 that return the sliders 5012, 5014 to a retracted position are considered within the scope of this disclosure. The biasing member 5030 can be any elastic material that allows for biasing the sliders to the retracted configuration. For example, the biasing member 5030 can be made of a rubber, plastic or metal.

Figure 51 illustrates the upper jaw 5010 and lower jaw 5011 in a closed configuration in which the upper jaw 5010 and lower jaw 5011 substantially abut one another. As seen in the cross-sectional view, the closing mechanism 5022 is configured to be mounted within a groove 5020. The closing mechanism 5022 can be glued or otherwise affixed within the groove 5020. In yet other embodiments, the groove 5020 can be omitted and the closing mechanism 5022 can be tapered such that the outer edges of the closing mechanism 5022 are substantially flush with the outer surface of the upper jaw 5010 and lower jaw 5011.

As illustrated in Figure 51, the biasing members 5030 are shown in their retracted configuration.

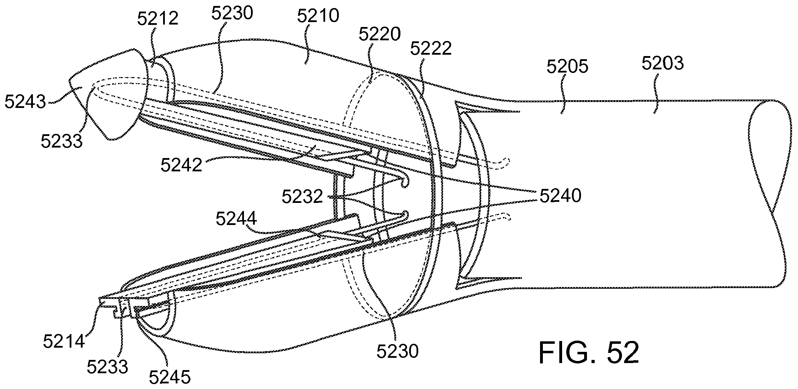

Figure 52 illustrates an exemplarily tip assembly 5205. The exemplarily tip assembly 5205 includes jaws 5210, 5212. An upper jaw 5210 can be configured to flex relative to the body 5203 of the tip assembly 5205. A lower jaw 5211 can also be configured to flex relative to the body 5203. The upper jaw 5210 can include a first slider 5212 that is slidingly coupled to the upper jaw 5210. The slidingly coupling can be through a one or more channels formed on the first slider; the one or more channels can be configured to matingly engage and slide relative corresponding receiving portions formed in the upper jaw 5212. In other embodiments, other configurations of the first slider 5212 and upper jaw 5210 are possible to allow relative motion of the first slider 5212 to the upper jaw 5210. The lower jaw 5211 can include a second slider 5214 that is slidingly coupled to the lower jaw 5211. The slidingly coupling can be through a one or more channels formed on the second slider; the one or more channels can be configured to matingly engage and slide relative corresponding receiving portions formed in the lower jaw 5211. In other embodiments, other configurations of the second slider 5214 and lower jaw 5211 are possible to allow relative motion of the second slider 5214 to the lower jaw 5211.

The upper jaw 5210 and lower jaw 5211 can be configured to flex relative to the body 5203, when a camera or other instrument abuts the upper jaw 5210 and lower jaw 5211. The first slider 5212 can have a wiper 5240 affixed to a first end 5242. The second slider 5214 can have a wiper 5240 affixed to a first end 5244. Thus, as the instrument abuts the wipers 5240, the upper jaw 5210 can separate from the lower jaw 5211. As the upper jaw 5210 separates from the lower jaw 5211, the first slider 5212 moves outward from the upper jaw 5210 and the second slider 5214 moves outward from the lower jaw 5214, thereby forming an opening between the upper jaw 5210 and lower jaw 5211 through which the instrument can pass. The wipers 5240 can be configured as described herein.

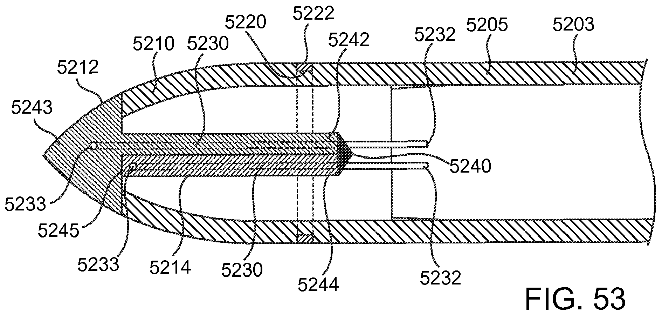

Additionally, a closing mechanism 5222 can be included in the tip assembly 5205. The closing mechanism 5222 can be configured to bias the upper jaw 5210 and lower jaw 5211 to a closed configuration as illustrated in the cross-sectional view of Figure 53. The closing mechanism 5222 in the illustrated embodiment is an elastic band. The elastic band can be metallic, plastic, or rubber. The elastic band can be configured to deliver a desired clamping force to the upper jaw 5210 and lower jaw 5211 so that the upper jaw 5210 and lower jaw 5211 close relative to one another to be in a closed position in which the upper jaw 5210 and lower jaw 5211 substantially abut one another. The closing mechanism 5222 can be configured to rest in a groove 5220 formed in the upper jaw 5210 and lower jaw 5211. The groove 5220 can be configured such that an outer surface of the closing mechanism 5222 is substantially flush with the outer surface of the upper jaw 5210 and lower jaw 5211.

The exemplarily tip assembly 5205 can further include slider biasing members 5230 that bias the first slider 5212 and the second slider 5214 to a retracted configuration relative to the upper jaw 5210 and lower jaw 5211, respectively. As illustrated the biasing member 5230 can be a single component for the first slider 5212 and a single component for the second slider 5214. In other embodiments, the biasing member 5230 can be made of one or more components. For instance, the first slider 5212 can have two biasing members. In yet other embodiments, the number of biasing members 5230 for the first slider 5212 can be one or any other number.

As illustrated the biasing member 5230 can include one or more attachment ends 5232 that are configured to be coupled to the body 5203. The attachment ends 5232 can be coupled to the body 5203 so that the biasing member 5230 can be releasably affixed to the body 5203. In yet other embodiments, the biasing member 5230 can be permanently affixed to the body 5203.

As illustrated the biasing member 5230 that passes through the first slider 5212 is configured to pass through a distal end 5243 of the first slider 5212 such that there is an distal portion 5233 of the biasing member 5230 that is nearest to the distal end 5243 of the first slider 5212. Similarly, the biasing member 5230 that passes through the second slider 5214 is configured to pass through a distal end 5245 of the second slider 5214 such that there is a distal portion 5233 of the biasing member 5230 that is nearest to the distal end 5245 of the second slider 5214. In other embodiments, the biasing member 5230 can have different configurations of a distal portion 5233 that can be further away from the distal end 5243, 5245 of the respective slider 5212, 5214. For example, the biasing member 5230 can run less than three-fourths the length of the slider 5212, 5214. In yet other embodiments the biasing member 5230 can run less than half the distance of the slider 5212, 5214. In still other embodiments, the length of the biasing member 5230 can be distance that is selected to provide the appropriate return force and allow for the appropriate opening so that the instrument can pass between the upper jaw 5210 and lower jaw 5211. As indicated above, the biasing member 5230 can be configured to return the sliders 5212, 5214 to a retracted position once the instrument has been removed. Other configurations of the biasing members 5230 that return the sliders 5212, 5214 to a retracted position are considered within the scope of this disclosure. The biasing member 5230 can be any elastic material that allows for biasing the sliders to the retracted configuration. For example, the biasing member 5230 can be made of a rubber, plastic or metal.

Figure 53 illustrates the upper jaw 5210 and lower jaw 5211 in a closed configuration in which the upper jaw 5210 and lower jaw 5211 substantially abut one another. As seen in the cross-sectional view, the closing mechanism 5222 is configured to be mounted within a groove 5220. The closing mechanism 5222 can be glued or otherwise affixed within the groove 5220. In yet other embodiments, the groove 5220 can be omitted and the closing mechanism 5222 can be tapered such that the outer edges of the closing mechanism 5222 are substantially flush with the outer surface of the upper jaw 5210 and lower jaw 5211.

As illustrated in Figure 53, the biasing members 5230 are shown in their retracted configuration. When the biasing members 5230 are coupled to the body 5203, the biasing members 5230 can provide a force to the first slider 5212 and second slider 5214 such that the sliders act to close the upper jaw 5210 and lower jaw 5211. While in the illustrated example the tip assembly 5205 includes the closing mechanism 5222, the closing mechanism 5222 can be omitted and the upper jaw 5210 and lower jaw 5211 can be biased to the closed configuration via the biasing members 5230.

Exemplary implementations have been described hereinabove regarding a jawed trocar assembly and a method of using the same. One of ordinary skill in the art will also appreciate that the elements and features illustrated in the implementations described and illustrated in the figures herein can be optionally included to achieve the benefits of the presently disclosed jawed trocar assembly. Additionally, those skilled in the art will appreciate that features in each of the figures described herein can be combined with one another and arrange to achieve the described benefits of the presently disclosed jawed trocar assembly. Various modifications to and departures from the disclosed implementations will occur to those having skill in the art. The subject matter that is intended to be within the scope of this disclosure is set forth in the following claims.

## Claims

1. A tip assembly comprising:
an adapter sleeve (205) configured to be coupled to a trocar;
a jaw assembly component coupled to the adapter sleeve, wherein the jaw assembly component comprises first and second jaws (511,515) hingedly coupled to an end of the adapter sleeve, each jaw being hingedly coupled to an outer portion of the end and each jaw being opposite to one another; the first jaw having a penetrating member (513) at the end thereof, the penetrating member adapted to penetrate at least one layer of body tissue; the second jaw hingedly coupled to the end of the adapter sleeve and opposite to the first jaw, **characterized by** a first slider (514) having the penetrating member at a distal end thereof, the first slider being coupled to the first jaw such that the first slider is axially translatable with respect to the first jaw; and
wherein the first slider comprises a first scope cleaner (705) coupled to a proximal end of the first slider opposite to the distal end of the first slider, the first scope cleaner being configured to sweep across an interior of the adapter sleeve when the first slider translates axially away from a proximal end of the first scope cleaner and the first jaw rotates radially away from the longitudinal axis thereby sweeping debris off of implements insertable into the adapter sleeve.

2. The tip assembly for a trocar as recited in claim 1, wherein the adapter sleeve and the jaw assembly component are integrally formed.

3. The tip assembly for a trocar as recited in claim 1, wherein the adapter sleeve and the jaw assembly are configured for mating engagement.

4. The tip assembly for a trocar as recited in any one of claims 1-3, wherein the adapter sleeve is configured to receive a first end of the trocar.

5. The tip assembly for a trocar as recited in any one of claims 1-4, wherein the adapter sleeve and jaw assembly component are substantially transparent allowing light to pass therethrough so that an image can be captured by a camera within the trocar assembly.

6. The tip assembly of claim 1, wherein in the first position, each of the jaws are substantially parallel to the longitudinal axis of the adapter sleeve and are positioned with respect to one another such that the ends of the jaws form a substantially conical contour adapted to penetrate the layer of the body tissue.

7. The tip assembly as recited in claim 6, wherein the pair of jaws comprises:
wherein in the first position, the first jaw and the second jaw are each substantially parallel to the longitudinal axis of the adapter sleeve and wherein the penetrating member of the first jaw extends longitudinally further than a distal end of the second jaw.

8. The tip assembly as recited in claim 7, wherein:
the second jaw comprises a second slider having a second scope cleaner coupled to a proximal end thereof and axially translatable with respect to the second jaw;
the second slider being coupled to the second jaw such that in the first position, the first scope cleaner and the second scope cleaner are adjacent one another; and
the second scope cleaner being configured to sweep across an interior of the adapter sleeve when the second slider translates axially away from the proximal end of the first scope cleaner.

9. The tip assembly as recited in claim 7 further comprising a biasing mechanism configured to bias the jaw assembly component in a second position.

10. A trocar assembly comprising:
a trocar; and
a tip assembly as recited in any one of claims 1-9 couplable to an end of the trocar.

11. The trocar assembly as recited in claim 10, wherein the scope cleaner is a wiper blade coupled to the jaw assembly component such that in the first position, the wiper blade is substantially adjacent to the end of the trocar.

12. The trocar assembly as recited in claim 11, wherein the wiper blade extends along a diameter of the end of the trocar.

13. An endoscopic tool assembly comprising:
a trocar assembly as recited in any one of claims 10-12; and
an endoscopic tool insertable through the trocar of the trocar assembly, the endoscopic tool being reciprocable within the trocar assembly.

14. The endoscopic tool assembly as recited in claim 13, wherein:
the first position comprises the endoscopic tool being received within the trocar assembly and advanced through the trocar assembly to a position where an end of the endoscopic tool is substantially flush with a distal end of the trocar assembly, and
the second position comprises the endoscopic tool being further advanced though the trocar assembly such that the end of the endoscopic tool protrudes beyond the distal end of the trocar assembly, thereby causing the jaw assembly component to rotate radially away from the longitudinal axis of the adapter sleeve until the endoscopic tool protrudes beyond the end of the jaw assembly component.

## Patentansprüche

1. Spitzenanordnung, die Folgendes umfasst:
eine Adapterhülse (205), die zur Verbindung mit einem Trokar konfiguriert ist;
eine Backenanordnungskomponente, die mit der Adapterhülse verbunden ist, wobei die Backenanordnungskomponente eine erste und zweite Backe (511,515) umfasst,
die aufklappbar mit einem Ende der Adapterhülse verbunden sind, wobei jede Backe aufklappbar mit einem äußeren Teil des Endes verbunden ist und jede Backe der anderen gegenüberliegt;
die erste Backe ein durchdringendes Element (513) am Ende davon aufweist, wobei das durchdringende Element zum Durchdringen von mindestens einer Schicht von Körpergewebe geeignet ist;
die zweite Backe aufklappbar mit dem Ende der Adapterhülse verbunden ist und der ersten Backe gegenüberliegt, **gekennzeichnet durch**
einen ersten Schieber (514), der das durchdringende Element an einem distalen Ende davon aufweist, wobei der erste Schieber mit der ersten Backe derart verbunden ist, dass der erste Schieber in Bezug auf die erste Backe axial beweglich ist; und
wobei der erste Schieber einen ersten Endoskopreiniger (705) umfasst, der mit einem proximalen Ende des ersten Schiebers, gegenüberliegend zum distalen Ende des ersten Schiebers, verbunden ist, wobei der erste Endoskopreiniger zum Abstreichen über ein Inneres der Adapterhülse konfiguriert ist, wenn der erste Schieber sich axial von einem proximalen Ende des ersten Endoskopreinigers wegbewegt und die erste Backe sich radial von der Längsachse wegdreht, wodurch Ablagerungen von in die Adapterhülse einführbaren Instrumenten abgestrichen werden.

2. Spitzenanordnung für einen Trokar nach Anspruch 1, wobei die Adapterhülse und die Backenanordnungskomponente integral ausgebildet sind.

3. Spitzenanordnung für einen Trokar nach Anspruch 1, wobei die Adapterhülse und die Backenanordnung für passendes Eingreifen konfiguriert sind.

4. Spitzenanordnung für einen Trokar nach einem der Ansprüche 1-3, wobei die Adapterhülse zur Aufnahme eines ersten Endes des Trokars konfiguriert ist.

5. Spitzenanordnung für einen Trokar nach einem der Ansprüche 1-4, wobei die Adapterhülse und Backensanordnungskomponente im Wesentlichen transparent sind, um Licht dahindurch zu lassen, sodass mit einer Kamera innerhalb der Trokaranordnung ein Bild aufgenommen werden kann.

6. Spitzenanordnung nach Anspruch 1, wobei in der ersten Position die Backen jeweils im Wesentlichen parallel zur Längsachse der Adapterhülse liegen und in Bezug aufeinander derart positioniert sind, dass die Enden der Backen eine im Wesentlichen konische Kontur bilden, die geeignet ist, die Schicht des Körpergewebes zu durchdringen.

7. Spitzenanordnung nach Anspruch 6, wobei das Paar von Backen Folgendes umfasst:
wobei in der ersten Position die erste Backe und die zweite Backe jeweils im Wesentlichen parallel zur Längsachse der Adapterhülse liegen und wobei das durchdringende Element der ersten Backe sich längs weiter erstreckt als ein distales Ende der zweiten Backe.

8. Spitzenanordnung nach Anspruch 7, wobei:
die zweite Backe einen zweiten Schieber umfasst, der einen zweiten Endoskopreiniger aufweist, der mit einem proximalen Ende davon verbunden ist, und in Bezug auf die zweite Backe axial beweglich ist;
der zweite Schieber mit der zweiten Backe derart verbunden ist, dass in der ersten Position der erste Endoskopreiniger und der zweite Endoskopreiniger nebeneinander liegen; und
der zweite Endoskopreiniger zum Abstreichen über ein Inneres der Adapterhülse konfiguriert ist, wenn der zweite Schieber sich axial von einem proximalen Ende des ersten Endoskopreinigers wegbewegt.

9. Spitzenanordnung nach Anspruch 7, die weiter einen Vorspannmechanismus umfasst, der zum Vorspannen der Backenanordnungskomponente in einer zweiten Position konfiguriert ist.

10. Trokaranordnung, die Folgendes umfasst:
einen Trokar; und
eine Spitzenanordnung nach einem der Ansprüche 1-9, die mit einem Ende des Trokars verbindbar ist.

11. Trokaranordnung nach Anspruch 10, wobei der Endoskopreiniger ein Wischerblatt ist, das mit der Backenanordnungskomponente derart verbunden ist, dass in der ersten Position das Wischerblatt im Wesentlichen benachbart zum Ende des Trokars vorliegt.

12. Trokaranordnung nach Anspruch 11, wobei das Wischerblatt sich entlang eines Durchmessers des Endes des Trokars erstreckt.

13. Endoskopische Instrumentanordnung, die Folgendes umfasst:
eine Trokaranordnung nach einem der Ansprüche 10-12; und
ein endoskopisches Instrument, das durch den Trokar der Trokaranordnung einführbar ist, wobei das endoskopische Instrument innerhalb der Trokaranordnung hin- und herbewegbar ist.

14. Endoskopische Instrumentanordnung nach Anspruch 13, wobei:
die erste Position umfasst, dass das endoskopische Instrument innerhalb der Trokaranordnung aufgenommen wird und durch die Trokaranordnung zu einer Position vorgeschoben wird, wo ein Ende des endoskopischen Instruments im Wesentlichen mit einem distalen Ende der Trokaranordnung bündig ist, und
die zweite Position umfasst, dass das endoskopische Instrument weiter durch die Trokaranordnung vorgeschoben wird, sodass das Ende des endoskopischen Instruments über das distale Ende der Trokaranordnung hinausragt, wodurch bewirkt wird, dass die Backenanordnungskomponente sich radial von der Längsachse der Adapterhülse wegdreht bis das endoskopische Instrument über das Ende der Backenanordnungskomponente hinausragt.

## Revendications

1. Ensemble pointe comportant :
un manchon adaptateur (205) configuré pour être accouplé à un trocart ;
un composant d'ensemble mâchoires accouplé au manchon d'adaptateur, dans lequel le composant d'ensemble mâchoires comporte des première et seconde mâchoires (511, 515) accouplées par charnière à une extrémité du manchon d'adaptateur, chaque mâchoire étant accouplée par charnière à une partie externe de l'extrémité et chaque mâchoire étant à l'opposé l'une de l'autre ;
la première mâchoire présentant un élément de pénétration (513) au niveau de son extrémité, l'élément de pénétration étant conçu pour pénétrer dans au moins une couche d'un tissu corporel ;
la seconde mâchoire accouplée par charnière à l'extrémité du manchon d'adaptateur et à l'opposé de la première mâchoire, **caractérisé par**,
un premier volet à coulisse (514) présentant l'élément de pénétration au niveau d'une extrémité distale de celui-ci, le premier volet à coulisse étant accouplé à la première mâchoire de telle sorte que le premier volet à coulisse est déplacé axialement en translation par rapport à la première mâchoire ; et
dans lequel le premier volet à coulisse comporte un premier nettoyeur d'appareil (705) accouplé à une extrémité proximale du premier volet à coulisse à l'opposé de l'extrémité distale du premier volet à coulisse, le premier nettoyeur d'appareil étant configuré pour balayer un espace intérieur du manchon d'adaptateur lorsque le premier volet à coulisse se déplace axialement en translation de façon à s'éloigner d'une extrémité proximale du premier nettoyeur d'appareil et la première mâchoire effectue une rotation radiale de façon à s'éloigner de l'axe longitudinal, balayant ainsi les débris des instruments pouvant être insérés dans le manchon d'adaptateur.

2. Ensemble pointe destiné à un trocart selon la revendication 1, dans lequel le manchon d'adaptateur et le composant d'ensemble mâchoires sont formés d'un seul tenant.

3. Ensemble pointe destiné à un trocart selon la revendication 1, dans lequel le manchon d'adaptateur et l'ensemble mâchoires sont configurés pour se mettre en prise de manière appariée.

4. Ensemble pointe destiné à un trocart selon l'une quelconque des revendications 1 à 3, dans lequel le manchon d'adaptateur est configuré pour recevoir une première extrémité du trocart.

5. Ensemble pointe destiné à un trocart selon l'une quelconque des revendications 1 à 4, dans lequel le manchon d'adaptateur et le composant d'ensemble mâchoires sont sensiblement transparents, permettant à la lumière de les traverser de façon à ce qu'une image puisse être saisie par une caméra à l'intérieur de l'ensemble trocart.

6. Ensemble pointe selon la revendication 1, dans lequel dans la première position, chacune des mâchoires est sensiblement parallèle à l'axe longitudinal du manchon d'adaptateur et est positionnée l'une par rapport à l'autre de telle sorte que les extrémités des mâchoires forment un contour sensiblement conique conçu pour pénétrer dans la couche de tissu corporel.

7. Ensemble pointe selon la revendication 6, dans lequel la paire de mâchoires comporte :
lorsque dans la première position, la première mâchoire et la seconde mâchoire sont chacune sensiblement parallèles à l'axe longitudinal du manchon d'adaptateur et dans lequel l'élément de pénétration de la première mâchoire s'étend longitudinalement plus loin qu'une extrémité distale de la seconde mâchoire.

8. Ensemble pointe selon la revendication 7, dans lequel :
la seconde mâchoire comporte un second volet à coulisse présentant un second nettoyeur d'appareil accouplé à une extrémité proximale de celui-ci et pouvant se déplacer axialement en translation par rapport à la seconde mâchoire ;
le second volet à coulisse étant accouplé à la seconde mâchoire de telle sorte que dans la première position, le premier nettoyeur d'appareil et le second nettoyeur d'appareil sont adjacents l'un par rapport à l'autre ; et
le second nettoyeur d'appareil étant configuré pour balayer un espace intérieur du manchon d'adaptateur lorsque le second volet à coulisse se déplace axialement en translation de façon à s'éloigner de l'extrémité proximale du premier nettoyeur d'appareil.

9. Ensemble pointe selon la revendication 7 comportant en outre un mécanisme de sollicitation configuré pour solliciter le composant d'ensemble mâchoires dans une seconde position.

10. Ensemble trocart comportant :
un trocart ; et
un ensemble pointe selon l'une quelconque des revendications 1 à 9 pouvant être accouplé à une extrémité du trocart.

11. Ensemble trocart selon la revendication 10, dans lequel le nettoyeur d'appareil est une lame d'essuyage accouplée au composant d'ensemble mâchoires de telle sorte que dans la première position, la lame d'essuyage est sensiblement adjacente à l'extrémité du trocart.

12. Ensemble trocart selon la revendication 11, dans lequel la lame d'essuyage s'étend le long d'un diamètre de l'extrémité du trocart.

13. Ensemble outil endoscopique comportant :
un ensemble trocart selon l'une quelconque des revendications 10 à 12 ; et
un outil endoscopique pouvant être inséré dans le trocart de l'ensemble trocart, l'outil endoscopique pouvant effectuer un mouvement de va-et-vient à l'intérieur de l'ensemble trocart.

14. Ensemble outil endoscopique selon la revendication 13, dans lequel :
la première position comporte l'outil endoscopique qui est reçu à l'intérieur de l'ensemble trocart et avancé dans l'ensemble trocart jusqu'à une position à laquelle une extrémité de l'outil endoscopique est sensiblement dans l'alignement d'une extrémité distale de l'ensemble trocart, et
la seconde position comporte l'outil endoscopique qui est avancé davantage dans l'ensemble trocart de telle sorte que l'extrémité de l'outil endoscopique fait saillie au-delà de l'extrémité distale de l'ensemble trocart, amenant ainsi le composant d'ensemble mâchoires à effectuer une rotation radiale de façon à s'éloigner de l'axe longitudinal du manchon d'adaptateur jusqu'à ce que l'outil endoscopique fasse saillie au-delà de l'extrémité du composant d'ensemble mâchoires.
